# EUROPEAN PATENT SPECIFICATION

(11) **EP 3 371 691 B1**
(45) Date of publication and mention of the grant of the patent: **11.03.2026**
(21) Application number: 16862929.3
(22) Date of filing: 03.11.2016
(51) Int. Cl.: G06T 7/11, G16B 25/00, G16B 45/00, G06T 7/187, G06T 7/194

(54) **SYSTEMS AND METHODS FOR PROCESSING SPATIALLY RELATED SEQUENCE DATA RECEIVED FROM A SEQUENCING DEVICE**
SYSTEME UND VERFAHREN ZUR VERARBEITUNG VON RÄUMLICH ASSOZIIERTEN SEQUENZDATEN VON EINER SEQUENZIERUNGSVORRICHTUNG
SYSTÈMES ET PROCÉDÉS DE TRAITEMENT DE DONNÉES DE SÉQUENCE LIÉES SPATIALEMENT ET REÇUES D'UN DISPOSITIF DE SÉQUENÇAGE

(30) Priority: 03.11.2015 US 201562250255 P
(43) Date of publication of application: 12.09.2018
(73) Proprietor: President and Fellows of Harvard College, Cambridge, MA 02138 (US)
(72) Inventor: DAUGHARTHY, Evan R., Cambridge, Massachusetts 02139 (US); DASARI, Vivek, Cambridge, Massachusetts 02139 (US); CHURCH, George M., Brookline, Massachusetts 02446 (US)
(74) Representative: J A Kemp LLP
(86) International application number: PCT/US2016/060243
(87) International publication number: WO 2017/079382

(56) References cited:
- WO-A1-2014/163886
- US-A1- 2006 248 349
- US-A1- 2007 087 362
- US-A1- 2010 223 276
- VIVEK DASARI: "Platform for Spatial Molecular Data by Vivek Dasari 1-7 Sig nature redacted Thesis Supervisor", 20 August 2015 (2015-08-20), XP055559164, Retrieved from the Internet <URL:https://dspace.mit.edu/bitstream/handle/1721.1/107103/971494098-MIT.pdf?sequence=1> [retrieved on 20190220]
- NG L ET AL: "Surface-based mapping of gene expression and probabilistic expression maps in the mouse cortex", METHODS, ACADEMIC PRESS, NL, vol. 50, no. 2, 1 February 2010 (2010-02-01), pages 55 - 62, XP026857255, ISSN: 1046-2023, [retrieved on 20091008]

## Description

### STATEMENT REGARDING FEDERALLY SPONSORED RESEARCH

This invention was made with Government support under P50 HG005550 awarded by The National Institutes of Health. The Government has certain rights to this invention.

### TECHNICAL FIELD

Aspects of the disclosure relate to electronic devices and computer software for processing spatially related sequence data received from a sequencing device. For example, some aspects of the disclosure relate to analyzing and processing data obtained from light intensity values obtained from fluorescence or optically encoded nucleic acid sequencing methods.

### BACKGROUND

Since many gene products such as ribonucleic acid (RNA) and proteins are enriched in regions where they function, their location may provide an important clue as to their function. This property has been used for in situ fluorescent hybridization, immunohistochemistry and tissue-specific reporter assays in numerous areas of biological research. Optical sequencing methods may provide light emanating from a sample which can be optically detected.

The need for multiplexing in biology and detection in general is driven by the fact that most systems are composed of a large number of unique species of molecules and are highly heterogeneous in composition. Together, these factors cause the combinatorial space of molecular organization to be very large. Thus, it may be difficult to detect, identify, count, quantify, or segment both large numbers of molecular components and their configurations.

WO 2014/163886 A1 discloses methods of making a three-dimensional matrix of nucleic acids within a cell.

NG L ET AL: "Surface-based mapping of gene expression and probabilistic expression maps in the mouse cortex", METHODS, ACADEMIC PRESS, NL, vol. 50, no. 2, 1 February 2010 (2010-02-01), pages 55-62, discloses a methodology for constructing surface-based flatmaps of the mouse cortex that enables mapping of gene expression data from individual genes in the Allen Brain Atlas, or probabilistic expression maps from the Anatomic Gene Expression Atlas, to identify and visualize genetic relationships between layers and areas.

### SUMMARY

The scope of the invention is defined in the appended claims. Any "aspect", "example" and "embodiment" of the description not falling within the scope of the claims does not form part of the invention and is provided for illustrative purposes only.

Aspects of the disclosure provide effective, efficient, scalable, and convenient technical solutions that address and overcome the technical problems associated with detecting, identifying, counting, quantifying, and/or segmenting molecular components and their configurations. In particular, one or more aspects of the disclosure provide techniques for processing spatially related sequence data received from a sequencing device.

In accordance with one or more embodiments, a computer system having at least one processor and memory may receive, from a sequencing device, image data associated with a sample. Subsequently, the system may identify, based on the image data received from the sequencing device, a first sequence located at first spatial coordinates. Then, the system may store, in a spatially searchable database, a first data element comprising the first spatial coordinates and a first identifier corresponding to the first sequence to spatially relate the first sequence to other sequences present in the sample.

In some embodiments, the system may identify, based on the image data received from the sequencing device, a second sequence located at second spatial coordinates. Subsequently, the system may store, in the spatially searchable database, a second data element comprising the second spatial coordinates and a second identifier corresponding to the second sequence to spatially relate the second sequence to the other sequences present in the sample.

In some embodiments, the image data received from the sequencing device may include spatial information, temporal information, and color information associated with the sample.

In some embodiments, the system may present, on a display device, information identifying a presence of the first sequence at the first spatial coordinates.

In some embodiments, the system may determine one or more metrics associated with the first sequence at the first spatial coordinates. In addition, the system may present, on a display device, information identifying the one or more metrics associated with the first sequence at the first spatial coordinates.

In some embodiments, the system may perform a linked annotations query on the spatially searchable database to obtain annotation query results information. In addition, the system may present, on a display device, at least a portion of the annotation query results information.

In some embodiments, the system may perform a spatial query on the spatially searchable database to obtain spatial query results information. In addition, the system may present, on a display device, at least a portion of the spatial query results information. In some instances, the system may receive user input requesting the spatial query, and the spatial query may be performed in response to receiving the user input requesting the spatial query.

In some instances, performing the spatial query on the spatially searchable database may include jointly querying spatial coordinates data and linked annotations data maintained in the spatially searchable database. In some instances, performing the spatial query on the spatially searchable database may include performing a three-dimensional distance query to identify a three-dimensional distance between the first sequence and another sequence present in the sample. In some instances, performing the spatial query on the spatially searchable database may include performing a containment query to identify a position of the first sequence relative to other features of the sample.

In some embodiments, the spatially searchable database may be maintained in a geographic information system (GIS) format. In some embodiments, the sequencing device may be configured to sequence the sample using fluorescent in situ sequencing (FISSEQ).

These features, along with many others, are discussed in greater detail below.

### BRIEF DESCRIPTION OF THE DRAWINGS

The present disclosure is illustrated by way of example and not limited in the accompanying figures in which like reference numerals indicate similar elements and in which:
FIG. 1 depicts an illustrative example of a spatial molecular data system which receives data from a sequencing device in accordance with one or more example embodiments;
FIGS. 2-15 depict illustrative examples of processing spatially related sequence data received from a sequencing device in accordance with one or more example embodiments;
FIGS. 16A and 16B depict an illustrative computing environment for processing spatially related sequence data received from a sequencing device in accordance with one or more example embodiments;
FIGS. 17A-17E depict an illustrative event sequence for processing spatially related sequence data received from a sequencing device in accordance with one or more example embodiments; and
FIG. 18 depicts an illustrative method for processing spatially related sequence data received from a sequencing device in accordance with one or more example embodiments.

### DETAILED DESCRIPTION

In the following description of various illustrative embodiments, reference is made to the accompanying drawings, which form a part hereof, and in which is shown, by way of illustration, various embodiments in which aspects of the disclosure may be practiced. In addition, it is noted that various connections between elements are discussed in the following description. It is noted that these connections are general and, unless specified otherwise, may be direct or indirect, wired or wireless, and that the specification is not intended to be limiting in this respect.

Some aspects of the disclosure relate to the analysis and processing of spatial molecular data. Spatial molecular data refers to data representing the identity and spatial organization of molecules which can result from the multiplex measurement, simultaneous or serial, co-registration in physical or digital space, of the identity and spatial organization of molecules. According to one or more aspects of the present disclosure, spatial molecular data can be obtained using methods such as fluorescent in situ sequencing (FISSEQ) and other known methods for detecting, sequencing, identifying, measuring, counting, quantifying, and/or segmenting molecules, detection reagents, or analytes in a sample in a multiplex manner.

According to aspects of the present disclosure, space refers to N-dimensional space (e.g. one-dimensional or 1D, two-dimensional or 2D, three-dimensional or 3D, four-dimensional or 4D, or more), where the spatial dimensions may refer to absolute Cartesian space, e.g. XYZT in absolute physical space (e.g. in units of angstroms, nanometers, microns, seconds, etc.); relative spatial relationships, e.g. tissue sections or biopsies (which are related in space but the absolute spatial relationship might not be known); or abstract spatial relationships, such as different organisms or experiments (which are related in space by virtue of existing in physical space, but have no meaningful spatial relationship other than not existing in the same space), which can still be meaningfully indexed and searched.

The practice of the methods disclosed herein may employ biology methods, software, computers and computer systems. Accordingly, one or more of the methods described herein may be computer implemented methods in whole or in part. Computer software utilized in the methods of the present disclosure includes computer readable media having computer-executable instructions for performing logic steps of the disclosed methods. Suitable computer readable media include, but are not limited to, a floppy disk, CD-ROM/DVD/DVD-ROM, hard-disk drive, flash memory, ROM/RAM, magnetic tapes, and others that may be developed. The computer executable instructions may be written in a suitable computer language or combination of several computer languages. The methods described herein may also make use of various computers and computer program products and software for a variety of purposes including obtaining and processing light intensity into data values, storage of light intensity data and other methods and aspects described herein including implementing one or more of the computational methods or systems or features described herein.

Computational methods within the scope of the present disclosure include post-acquisition processing, storage, annotation, management, and analysis of raw optical data, such as that obtained from a FISSEQ protocol using optical detectors to obtain the light intensity data. Computational methods within the scope of the present disclosure include analysis, storage, annotation, management, and post-processing of the initial analysis from raw optical data, such as that obtained from a FISSEQ protocol using optical detectors to obtain the light intensity data. Computational methods within the scope of the present disclosure include extraction, processing, storage, annotation, management, and analysis of molecular features, aspects or qualities of molecular features, or biological features annotated by one or more molecular features, in space.

Computational methods within the scope of the present disclosure include establishment, initiation, annotation, management, tracking, updating, mapping, and inter-converting, for a relative or absolute coordinate space for spatial molecular data. Computational methods within the scope of the present disclosure include linking different types of spatial molecular data and linking spatial molecular data with image data. Computational methods within the scope of the present disclosure include spatial co-registration, digital spatial representation, tracking, and static or dynamic management, of different types of spatial molecular data and/or spatial molecular data with image data. Computational methods within the scope of the present disclosure include visualization, interaction, query of spatial molecular data, biological and molecular data, image data, coordinate spatial information, and analysis methods and analysis products.

Aspects of the present disclosure include an image service, an object service, or an application service. Aspects of the present disclosure include an API for interfacing with a spatial molecular data repository. Aspects of the present disclosure include an API for interfacing with independent analytical tools. Aspects of the present disclosure include methods for querying, receiving, storing, and analyzing external annotation sources of molecular data e.g. NCBI, Gene Ontology Consortium, etc. Aspects of the present disclosure include methods of using a database for storage, tracking, access, computation, updating, mapping, managing, and processing spatial molecular data, particularly raw signals, processed signals, and digitized or vectorized signals. Aspects of the present disclosure include methods of using a geographic information system (GIS) database for processing or manipulating spatial molecular data, where digitized or vectorized signals specifically refer to geometric (GEOM) objects such as points, lines, triangles, polygons, shells, volumes, masks, meshes, graphs, trajectories, etc.

According to certain aspects, spatial molecular data may be obtained from imaging light intensity from a three dimensional matrix of nucleic acid sequences using methods described and disclosed in PCT/US2014/18580. Useful methods also include immobilizing naturally occurring nucleic acids within their native environment, such as within a cell or within a tissue sample. The three dimensional nucleic acid matrix may be generated in situ in a cell or tissue sample to preserve the naturally occurring nucleic acid sequence diversity (such as DNA and RNA) and spatial orientation in cells, tissues or any other complex biomaterial. In these instances, the location of nucleic acids and their relative position may be identified as a three dimensional structure, such as within subcellular compartments, within cells, within tissues, as three dimensional nucleic acid assemblies, as three dimensional nucleic acid material, etc. The nucleic acids may be amplified and sequenced, if desired, in situ thereby providing positional information of the nucleic acids within the cell or tissue.

The present disclosure provides methods, such as computer hardware and software implemented methods of analyzing spatial molecular data. The present disclosure also provides a system of various services or functions utilizing, analyzing, or processing spatial molecular data using, for example, computer hardware and software, which may be referred to herein as a spatial molecular data system. The spatial molecular data system is used to store, visualize, and compute on, image data representing molecular species or molecular features. The image data may be obtained from optical sequencing methods such as FISSEQ (sequencing data) or other types of optical detection of molecular species or molecular features such as stains, immunohistochemistry, etc. According to certain aspects, molecular data is extracted from the image data and processed into sets of fundamentally linked geometries and molecular annotations. The geometries are points, lines, polygons, meshes, surfaces, etc., and are conveniently and efficiently stored in a GIS database in a format such as "well known binary." The linked molecular annotations are sequences, sequence alignments, SNPs, genomic loci, proteins, modifications, complexes, etc. "3D Render" describes the process of extracting the geometries from image data, with the exception of nucleic acid sequencing data (endogenous RNA, DNA, or synthetic barcodes), which are extracted separately as FASTA or SAM/BAM format sequences linked to or identified by 3D spatial coordinates. The GIS provides for spatial queries of the data and spatial computations such as 3D distance and containment (within). Software services interface the GIS with tools such as statistical computing, e.g. to calculate things like local density. Software services interface with bioinformatic databases and bioinformatic methods, e.g. to calculate things like differences in gene expression. Software services interface with the statistical computing and bioinformatic databases and methods, e.g. to calculate things like de novo 3D spatial segmentation based on gene expression patterns. Software services such as a client provide for web-browser-based visualization of 3D representations of the geometries with visualization tools to select subsets of the data based on the molecular annotations, e.g. to display only geometries of a certain molecular species. A command line interface provides for user interaction with a set of software APIs for GIS computation, statistical computing, bioinformatic computations, and visualization. Ideally, the image data the molecular geometries were extracted from are co-linked in space with the molecular geometries where the image data is retained, such that given a spatial coordinate of a molecular geometry is it possible to retrieve the corresponding source image data, as from an image server software service. In order to achieve a homogenous "data universe" of image data and molecular geometries over large physical spatial domains, software services maintain and enforce a coordinate system underlying the data representation. The coordinate system is ideally a Cartesian grid representing physical space in units such as angstroms or nanometers. The coordinate system service performs essential tasks such as defining the boundaries of the data universe, extending the data universe to accommodate new data within an experiment (e.g. new sections of a serial section for the purpose of volumetric reconstruction), and homogenization (e.g. if some data is acquired at a different resolution or magnification than other data).

Aspects of the disclosure are further illustrated by the following examples, which should not be construed as limiting.

### EXAMPLE I: Spatial Molecular Data System

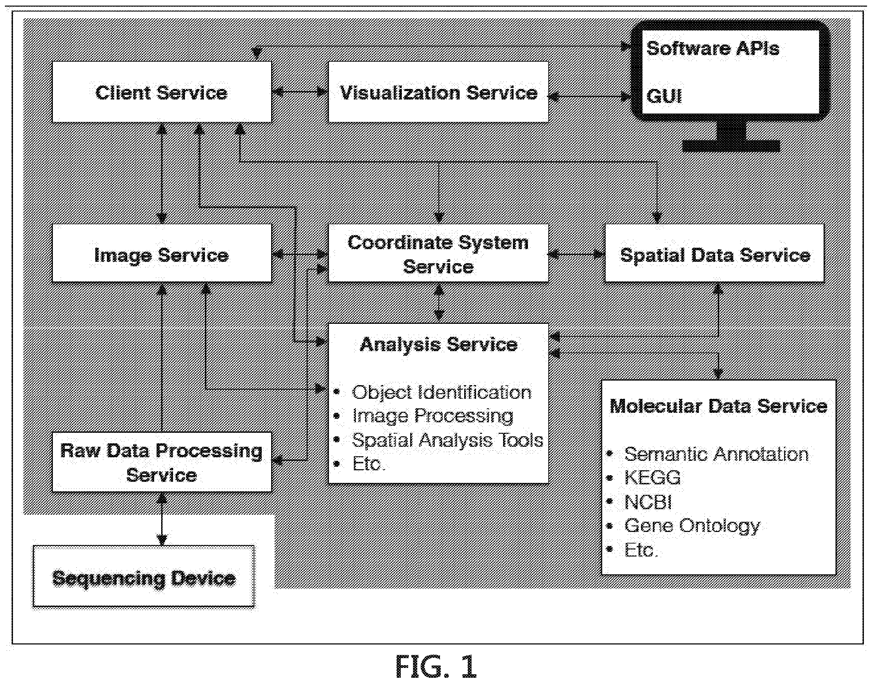

A Spatial Molecular Data System of the present disclosure is described with reference to FIG. 1, which is a schematic of an illustrative system as a whole with the various components and how each component may interface with one or more other components. It is to be understood that arrowed lines indicate an interface between the components connected by the arrowed lines using a suitable software and/or hardware interface.

The Spatial Molecular Data System (SMDS) is a connected and integrated set of services, including all or some of the following services: Raw Data Stream Handling Services; Spatial Coordinate System Services; Object Identification Services; Image Services; Object Services; Data Services; Analysis Services or Visualization Services. The term service refers to a set of related software functionalities that carry out a particular function or set of functions, and can be used or reused for any suitable purpose, together with the policies that formalize the service's use.

The present disclosure describes a set of software functionalities, as well as organization of these software functionalities into services. The present disclosure also includes alternative organization of these software functionalities. The present disclosure also includes non-service-oriented architectures, such as Web-oriented architectures, that provide some or all of these software functionalities.

According to one aspect, a computer implemented system for processing spatial molecular data including a raw data processing service which receives raw data from a sequencing device, an image service for receiving, storing, and serving images, a coordinate system service that uses one or more numbers, or coordinates, to determine the position of geometric elements on a manifold, a spatial data service for storing, retrieving and processing of spatial data and spatial relationships, a molecular data service for receiving, updating, version tracking, managing and querying relational biological databases, an analysis service for providing access to data analysis packages, a visualization service initializing an N-dimensional space, constructing queries to request images from an image service and rendering the images for display, software APIs and a GUI, a client service, a developer interface, and a user interface. According to one aspect, the raw data processing service exchanges data with and is communicatively coupled to the image service and the coordinate system service. According to one aspect, the image service exchanges data with and is communicatively coupled to the raw data processing service, the client service, the coordinate system service and the analysis service. According to one aspect, the client service exchanges data with and is communicatively coupled to the image service, the visualization service, the software APIs and GUI, the coordinate system service, the spatial data service and the analysis service. According to one aspect, the visualization service exchanges data with and is communicatively coupled to client service and the software APIs and GUI. According to one aspect, the coordinate system service exchanges data with and is communicatively coupled to image service, the spatial data service, the analysis service, the client service, and the raw data processing service. According to one aspect, the software APIs and GUI exchanges data with and is communicatively coupled to the visualization service and the client service. According to one aspect, the analysis service exchanges data with and is communicatively coupled to the image service, the client service, the coordinate system, the spatial data service and the molecular data service. According to one aspect, the molecular data service exchanges data with and is communicatively coupled to the analysis service. According to one aspect, the spatial data service exchanges data with and is communicatively coupled to the client service, the coordinate system, and the analysis service.

### Sequencing Device

A sequencing device is indicated in FIG. 1 which provides raw data to a raw data processing service of the Spatial Molecular Data System (SMDS). According to one aspect, an automated sequencing and three-dimensional imaging device is provided which uses volumetric three dimensional imaging modalities to image a three dimensional nucleic acid matrix. The device includes hardware and software functionally assembled to enact a protocol of sequencing and imaging of the three dimensional nucleic acid containing matrix which is contained within a suitable vessel or stage. The device may be referred to as a fluidic sequencing microscope to the extent that it includes hardware and software to automate sequencing and hardware and software for volumetric imaging. A suitable sequencing device may be commercially available such as the I15 Polonator sequencing device available from Danaher Corporation. The I15 Polonator sequencing device can obtain two-dimensional image data or can be modified to obtain three-dimensional image data. The raw data obtained by the I15 Polonator device is transmitted to the raw data processing service.

### Raw Data Stream Processing or Handling Services

The raw data processing service is a services layer of SMDS including one or more physical or virtual computers. Each computer includes one or more physical or virtual memory-coupled processors and a software-based operating system which is local or networked connection to other SMDS components as indicated in FIG. 1. The raw data stream from the sequencing device is received by a computer system. The raw data stream may be stored locally (cached) or processed in real time, and/or further stored according to the data storage systems. The raw data stream contains pixel values. The raw data stream may also include metadata associated with the image. The raw data stream or image pixel data may be compressed (e.g. gzip, LZW, etc.) Metadata may include all experimental (e.g. cell type, experimenter, etc.) and acquisition (e.g. light sources, filters, detectors, magnification, exposure times, physical encoder values for N-dimensional, 1D, 2D, 3D, 4D, or more dimensions, etc.) metadata. The computer system, optionally through coordination with the sequencing device or a human user, may generate additional image metadata annotations or edit the metadata annotations generated by the sequencer device. The computer system may provide, or be linked to a processing service that provides, additional compression or decompression services for image data or metadata, as well as other image processing capabilities including sharpening, smoothing, filtering, thresholding, deconvolution, object identification, resampling, resizing, cropping, stitching, windowing, copying, or otherwise manipulating the image data. The computer system may provide feedback to the sequencing device. Feedback includes feedback to change image acquisition parameters, repeat imaging in part or whole, add additional sampling, change future sampling or image acquisition protocols, provide feedback on hardware or software offsets for dimensional encoders, stop the sequencing or trigger human user attention, repeat past or alter future sequencing chemistry protocols and steps, etc. Feedback may or may not be in real time. In general, the raw data processing service may receive, process, and store data and metadata from the sequencing device, user, and other software systems of the SMDS.

### Coordinate System Service

The coordinate system service is a services layer of SMDS including one or more physical or virtual computers. Each computer includes one or more physical or virtual memory-coupled processors and a software-based operating system which is local or networked connection to other SMDS components as indicated in FIG. 1. The coordinate system service is a system that uses one or more numbers, or coordinates, to determine the position of geometric elements on a manifold. The manifold is a topological space consisting of N-dimensions (e.g. 1D, 2D, 3D, 4D, 5 or more dimensions). The coordinate system may utilize either discrete or continuous coordinates representing absolute or relative spatial relationships, in Euclidean or non-Euclidean space. The spatial dimensions may refer to absolute Cartesian space, e.g. XYZT in absolute physical space (e.g. in units of angstroms, nanometers, microns, seconds, etc.); relative spatial relationships, e.g. tissue sections or biopsies (which are related in space but the absolute spatial relationship may not be known); or abstract spatial relationships, such as different organisms or experiments (which are related in space by virtue of existing in physical space, but have no meaningful spatial relationship other than not existing in the same space), which can still be meaningfully indexed and searched. In a particular implementation, the coordinate system is absolute spatial coordinates in units of angstroms, nanometers, or other absolute physical units, representing Euclidean space. In a particular implementation, the coordinate system utilizes a hybrid index using absolute, relative, and/or abstract spatial relationships. The coordinate system service is a service for instantiating, storing, maintaining, and updating a coordinate system. The coordinate system may be a global coordinate system containing all dimensions. The coordinate system service provides a method for instantiating, storing, maintaining, validating, checking, and updating spatial metadata values, spatial annotations, and/or coordinates of images, objects, etc. The coordinate system service may interface with and communicate with other services, including the Image services and Object services as shown in FIG. 1. The coordinate system service provides methods for homogenizing, converting, refactoring, standardizing, and computing spatial coordinates. The coordinate system service utilizes a method for receiving queries for the purpose of retrieving, instantiating, storing, maintaining, updating, homogenizing, converting, refactoring, standardizing, and computing spatial coordinates. The query may be in the form of a structured query. The coordinate system service provides methods for instantiating, storing, maintaining, and updating non-coordinate-based tracking of spatial relationships, such as parent-child relationships, and methods for communicating these relationships into metadata or features of data in Image Services or Spatial Data Services as shown in FIG. 1.

### Image Services

The image service is a services layer of SMDS including one or more physical or virtual computers. Each computer includes one or more physical or virtual memory-coupled processors and a software-based operating system which is local or networked connection to other SMDS components as indicated in FIG. 1. The image service performs methods of receiving, storing, and serving images and may organize large quantities of images. The image service consists of three core parts: controller, database, and static file storage and hosting. The image service is image format independent and supports commonly used image formats including TIFF, JPEG, Exif, PNG, RAW, etc. Static file storage refers to where and how the images are stored on disk or on a file system. Hosting these files as public assets allows them to be accessed through the web. Implementations of static file storage and hosting can either use AWS S3 storage and hosting (or any AWS competitor) or store and host the images locally. The database stores searchable metadata about each image along a way to access the image in memory (e.g. filename, URI, image data, etc.) Metadata includes coordinates provided by the coordinate system. Metadata includes versioning as indicated in Table 1 below which is a sample TIFF image schema for the image service database. The schema exposes important TIFF metadata for querying.

**TABLE 1**

| TIFF Images |
|---|
| Id: int PRIMARY KEY |
| Experimenter: string |
| XGlobal : int |
| YGlobal : int |
| ImageWidth : int |
| ImageLength : int |
| BitsPerSample : int |
| Compression : int (4 bit) |
| SamplesPerPixel : int |
| XResolution : int |
| YResolution : int |
| ResolutionUnit : int |
| DateTime : date |
| DocumentName : string |
| ImageDescription : text |
| Make : string |
| Model : string |
| Software : string |
| Exif IFD : string |
| LightSource : int |
| ColorSpace : int |

The image service performs a method for receiving queries and/or a software controller exposing an API, such as an exposed RESTful API to search and process images. The image service performs methods to return image data directly or reply with a URI to the resource. An example query is GET http://host.com/images?parameter1=this&parameter2=that. The imaging service performs methods for basic image processing (e.g. cropping, contrast modification, etc.) In a particular implementation, these features can be exposed by ImageMagick. The image service performs methods to generate and store mipmaps -- lower resolution representations of the same image -- of raw images that will be served at different zoom levels. This can be dynamically generated by the graphics card or manually preprocessed and stored. The image service performs methods to resample, stitch, register, calibrate, blend, and partition image data.

### Spatial Data Services

The spatial data service is a services layer of SMDS including one or more physical or virtual computers. Each computer includes one or more physical or virtual memory-coupled processors and a software-based operating system which is local or connected via a network to other SMDS components as indicated in FIG. 1. The spatial data service is responsible for the storage, retrieval, and processing of spatial data and spatial relationships. One implementation of a spatial data service may rely on the PostGIS extension for PostgreSQL database. The spatial data may be a spatially resolved annotation of molecular substrates, qualities or aspects of molecular substrates, or biological or cellular features labeled by one or more molecular substrates or qualities or aspects of molecular substrates including nucleic acid polymers including RNA and DNA; amino acid polymers including proteins; chemical modifications of any of the above; lipids, metabolites, biomolecules, and other small molecules; molecular compositions including one or more of any of the above; or cellular or biological features including organelles; membranes, nucleus, nucleolus, vacuoles, endoplasmic reticulum, Golgi, mitochondria, stress granules, P bodies, cytoskeleton, neurites or cellular projections, synapses, cilia and the like. Spatial data is any data that exists in the N-dimensional space. Examples of spatial data include mesh objects representing cell features or point objects representing sequencing reads. In the PostGIS implementation, spatial data is currently stored in the extended in well-known binary (EWKB) format. Spatial data operations performed by the image service return data relating to dimension, geometry type, ID, bounding box, binary representation, boundary of object, indication or check if object is empty, indication or check if object is simple. Spatial relationships supported by the service include equals, disjoint, intersects, touches, within, contains or overlaps. Spatial computations supported by the service include area, centroid, 3D distance, neighbor, features, density, and clustering. Spatial data may be annotated with one or more features, including a feature set including image-based features including, texture, size, angular moments, contrast, correlation, variance, difference moments, averages, variance, entropy, area, perimeter, form factor, solidity, extent, Euler number, centroid, eccentricity, major and minor axis lengths, orientation, compactness, maximum radius, median radius, mean radius, maximum and minimum feret diameters and Zernike shape features. Spatial data may be annotated with one or more features, including a feature set including sequence features including sequencing read, quality scores, one or more alignments to a reference sequence, e.g. stored in genomic coordinates, or relative to a structured reference file such as Refseq mRNA, alignment metadata, or link to alignment metadata and genomic loci (start, end, sequence). Spatial data may be annotated with one or more features, including a feature set including semantic or ontological features including biological annotations, cellular annotations and morphological annotations. Spatial data may be annotated with one or more features, including a feature set including FISSEQ measurement features.

FISSEQ describes a related suite of technologies characterized by in situ fluorescent sequencing. In Situ: Literally detection "in place," meaning within 1D, 2D, 3D, 4D, or N-dimensional space, such as within a gel, within cells, etc., as opposed to in solution. Fluorescent: Where the measured signal is fluorescence. Sequencing: Where the sequence or composition of a nucleic acid or other programmable polymer is determined, at least to the extent that the interrogated polymer does not have an equal probability of being any possible polymer. FISSEQ is a method whereby DNA is extended by adding a single type of fluorescently-labelled nucleotide triphosphate to the reaction, washing away unincorporated nucleotide, detecting incorporation of the nucleotide by measuring fluorescence, and repeating the cycle. At each cycle, the fluorescence from previous cycles is bleached or digitally subtracted or the fluorophore is cleaved from the nucleotide and washed away. FISSEQ is described further in Mitra et al. (2003) Anal. Biochem. 320:55. According to certain aspects, FISSEQ methods and materials useful in the practice of the methods described herein are provided in Lee et al., Nature Protocols, vol. 10, No. 3 (2015) pp. 442-458, Lee et al., Science 343, 1360-1363 (2014) and Supplementary Materials published 27 February 2014 on Science Express DOI: 10.1126/scienmce.1250212.

FISSEQ technologies can be decomposed into modular components. Molecular substrates describe what is being detected in situ such as nucleic acid polymers including RNA and DNA, amino acid polymers including proteins, chemical modifications of any of the above, lipids, metabolites, biomolecules, and other small molecules or molecular compositions including one or more of any of the above. FISSEQ can include identity encoding methods. These are (optional) methods or techniques for capturing information about the identity or an aspect or quality of a molecular substrate and converting the information content into a format amenable to amplification and/or detection. The encoding process must only have the quality of producing non-zero information content, e.g. such that the encoded product does not have an equal probability of indicating any possible molecular identity or molecular quality. Direct or inherent encoding refers to where some information about the identity or a particular aspect of the molecular substrate is inherently in a format amenable to amplification and/or detection. Reactivity-based encoding refers to where it is possible to capture information about the identity or a particular aspect or quality of a molecular substrate and convert the information to a format amenable to amplification and/or detection through reactivity with the substrate, including chemical, biochemical, and enzymatic, reactions. Such reactions include ligation of an adaptor to RNA or DNA, molecular inversion probe (MIP) capture or padlock/gap-fill capture of endogenous DNA/RNA sequence into a circular template, copying RNA or DNA sequencing information with modification, e.g. reverse transcription to generate cDNA or PCR to generate DNA containing a known primer region, terminal transferase to add untemplated bases to a RNA or DNA molecule, heat or enzymatic treatment to convert double-stranded genomic DNA to single-strand genomic DNA, chemical treatment of a protein to affect an antigen or change the state of a complex to enable a reaction, a reaction where an endogenous protein mediates a reaction which captures information about the protein, e.g. an endogenous ligase modifies an exogenous DNA molecule in situ or affinity-based encoding such as nucleic acid hybridization, affinity binding reagents, including antibody and aptamer binding and nucleoprotein/ribonucleoprotein binding (e.g. cas9, Argonaut family nucleic acid binding proteins). The product of these methods is referred to as the sequencing template.

The information content in FISSEQ is read out by means of optical detection of fluorescent signals, e.g. signal amplification methods. The following are examples of methods or techniques for generating an amplified fluorescent signal amenable to detection by various microscopic imaging modalities (which may have constraints on sensitivity to small numbers of photons, for example): No amplification, or single-molecule detection: Where it is possible to detect a fluorescent signal from a single molecule and a single fluorophore; Multiple labeling: Detection of the sequencing template using multiple fluorophores or fluorescent labels to generate a single signal, or to generate multiple signals which are combined using analog or digital means to achieve greater net signal; Photon accumulation: Accumulation of photons from one or more fluorophores, such as by periodically replacing the fluorophore (e.g. DNA PAINT) or stimulating emission, or other means to accumulate a sufficient number of photons for detection; Nucleic acid clonal amplification: Clonally amplifying a nucleic acid sequencing template, as by rolling circle amplification, polymerase chain reaction, in vitro transcription, etc., such that the sequencing or interrogation occurs in parallel across the clonal population; Hybridization chain reaction: Assembly of a localized nucleic acid nanostructure containing fluorophores; Information complexity reduction/probe tiling: Conversion of a complex but low-abundance template to a template with higher abundance but equal or less complexity, such as by tiling probes along a genomic loci that share a barcode; Hapten amplification: A molecule that can bind or react with another type of molecule in excess of 1:1 stoichiometric relationship, e.g. biotin which can bind many streptavidin proteins.

While encoding and amplification may be optional, sequencing is essential to FISSEQ. These methods describe all methods used to convert information content from the sequencing template into fluorescent signals for optical detection. In general, any method for determining the sequence or composition of a nucleic acid or other programmable polymer is determined, at least to the extent that the interrogated polymer does not have an equal probability of being any possible polymer. Exemplary methods include: Sequencing by hybridization: Nucleic acid polymer hybridization between two or more strands of DNA, cDNA, RNA, or other programmable polymer capable under certain conditions of annealing another such programmable polymer, such that some hybridization interactions are more favorable than others and therefore contain non-zero information content, and including the conditions under which such a process can be made arbitrarily specific, e.g. to 1-base, 2-base, or n-base mismatches; sequencing by nucleic acid synthesis: Templated synthesis of a nucleic acid such as DNA, cDNA, or RNA in any manner that generates fluorescent signals indicating the sequence or composition of the template, including reversible-terminator sequencing by synthesis (SBS, SolexalIllumina), sequencing by ligation (SBL, SOLiD), sequencing by reverse transcriptase (SBRT), etc.; sequencing by nucleoprotein/ribonucleoprotein binding: Interrogation of the sequence or composition of a template using a nucleoprotein or ribonucleoprotein capable of binding nucleic acids, such as Cas9; direct visualization: Direct visualization or imaging of the template to determine the sequence or composition; or affinity binding/protein binding: Interrogation of the sequence or composition of a template by affinity binding or protein binding, e.g. using a restriction enzyme or ssDNA binding protein such as RecA to read-out probed sequences.

Two common methods include barcoding and serial labeling. Both strategies involve repeat probing of the sample, while varying the probes used and/or the composition of the sample to increase the number of distinct labels. In the barcoding strategy, the combination of signals, typically distributed in space or over time, given by a sequencing template is used for identification. In the serial labeling strategy, the template is labeled, often reversibly, such that the signal is a priori known to be associated with the label. After detection of one or more templates, a new set of one or more templates can be labeled. The labeling may be additive, or previous labels may be removed before the addition of a new set of labels. In certain cases, the particular molecular species or configuration represented by the sequencing template may not be known, in which case this association may be discovered by the process of barcoded or serial labeling, or in the same manner associations between labels may be discovered.

Error detection/correction are examples of methods or techniques for enhancing the robustness of FISSEQ by adding some redundancy, or extra information, to the message beyond that which is necessary for determining the identity or an aspect or quality of a molecular substrate, in order to ether detect errors generating during identity encoding, amplification, or sequencing, or both detect errors and reconstruct the original error-free information (error detection and correction, respectively). Exemplary methods include: Error detection/correction of encoding: The encoding process may accumulate errors in many ways, such as synthesis errors for nucleic acid barcodes and enzymatic errors during template construction. Templates may be constructed such that additional information is encoded beyond that necessary for identification to effectively increase the Hamming distance between the encoded strings (e.g. constructing a cDNA template with 50 bases of endogenous RNA sequence where only 20 are needed to identify the RNA species). Other templates may be constructed to incorporate error detection and/or correction codes such as parity bits, checksums, Golay encoding, or any other method for detecting and/or correcting errors; Error detection/correction of reactivity or affinity: Errors can occur during encoding by reactivity or affinity where part of the information is lost (e.g. a reaction or affinity binding is not 100% complete) or erroneous (e.g. a reaction or affinity binding event is non-specific generating a potential false-positive). These types of errors can be detected and/or corrected again by using additional information. For example, the encoded information in the template plus additional information for error correction/detection may be divided among multiple probes such that a single probe in the absence of the other probes may be identified as an error, or so that the information may still be reconstructed even with some probes missing. Additional cross-probe information may also be encoded, e.g. signal from two probes could be known to be free of error if each probe encodes information about the other probe.

### Molecular Data Services

The molecular data service is a services layer of SMDS that may include one or more physical or virtual computers. Each computer includes one or more physical or virtual memory-coupled processors and a software-based operating system which is local or networked to other SMDS components as indicated in FIG. 1. The molecular data service provides or implements methods for receiving, updating, version tracking, managing, and querying, relational biological databases, including a database capable to store biological data; where the biological object model includes definitions for structure and function, genetics, biologic, expression objects, modification objects, molecular information, information about molecular variations, pathway information, and other types of biological information, and epistemological annotations (e.g. how the information is known, evidences, confidences, etc.); a database engine, communicatively coupled to the database, capable to search for and retrieve data from the database; a biological object model, communicatively coupled to the database engine, capable to store definitions for biological objects, the definitions capable to represent biological data as objects based on biological concepts, the biological objects each including at least one attribute, at least one behavior and at least one relationship to at least one other biological object; a data-mapping engine, communicatively coupled to the biological object model, capable to substantiate biological objects from retrieved data per the biological object model; a relational database of biological information comprising tables of biological data; a search module configured to receive a structured language query and convert said structured language query into a search statement for querying said relational database of biological information; a database graph generation module which creates a graph of said database; and a joins module configured to create optimized joins between said tables of biological data by utilizing said graph to calculate the shortest path between said tables specified in said query, said optimized joins being incorporated into said search statement by said search module for use in obtaining search results for a user. The molecular data service provides a relational database of biological databases, including schema objects, tables, data models, formats, and definitions. The molecular data service provides or implements methods for comparing versions of a database, such as where one version is local and one is not local and methods for tracking database status including changes and updates. The molecular data service provides or implements methods for receiving and responding to queries such as receiving a biological data retrieval request; retrieving the biological data corresponding to the request; substantiating the retrieved biological data as biological objects per a biological object model based on biological concepts, the biological objects each including at least one attribute, at least one behavior and at least one relationship to at least one other biological object; sending a structured language database query to a search engine; parsing a relational database and creating a database graph; creating correct joins between nodes corresponding to said query; translating said structured language database query into an SQL statement incorporating said correct joins; and sending said SQL statement to said relational database for obtaining search results for a user. The molecular data service provides or implements methods for processing, converting, homogenizing, and/or cross-referencing multiple biological databases. The molecular data service provides or implements a computer system for creation of at least one bioinformatics database. The molecular data service provides or implements a library of re-usable templates for establishing structure for the bioinformatics database. The molecular data service provides or implements a method for identifying a set of records in the plurality of biological/chemical databases that relates to a single biological/chemical object. The molecular data service provides or implements methods for establishing an entity in a data structure that corresponds to the single biological/chemical object, the entity including a plurality of aliases, a respective one of which refers to a respective record in the set of records in the plurality of biological/chemical databases. The molecular data service provides or implements methods of assigning a confidence level to at least one of the relationships in the entity-relationship model of the plurality of biological/chemical databases. The molecular data service provides or implements methods to establish, update, manage, track, and receive and respond to queries related to an ontological or semantic relational database. The term "ontology" refers to an explicit formal specification of how to represent objects, concepts and/or other entities that are assumed to exist in some area of interest, and the relationships that hold among such objects, concepts and/or other entities. One non-limiting example of an ontology is a hierarchical structuring of knowledge about things by subcategorizing them according to their essential (or at least relevant and/or cognitive) qualities.

### Analysis Services

The analysis service is a services layer of SMDS including one or more physical or virtual computers. Each computer includes one or more physical or virtual memory-coupled processors and a software-based operating system which is local or networked connection to other SMDS components as indicated in FIG. 1. The analysis service includes a server providing access to data analysis packages. The analysis service provides or implements methods of homogenization of software management through API-based software "wrappers" to provide for consistent management of inputs, outputs, parameters, error handling, and process management, including storing input and output requirements, conversion of inputs and outputs, receiving inputs and outputs, and handling errors. The analysis service provides or implements methods of scheduling analysis, building analysis pipelines from combinations of software, and mesh generation and format conversion.

### Visualization Services

The visualization service is a services layer of SMDS including one or more physical or virtual computers. Each computer includes one or more physical or virtual memory-coupled processors and a software-based operating system which is local or networked to other SMDS components as indicated in FIG. 1. The visualization service is responsible for initializing a virtual N-dimensional space. The space is populated with zero or more lighting objects, zero or more camera objects, zero or more images (rasterized or rendered as a volume), and zero or more annotation objects. An annotation object is any object existing in the N-dimensional space that is not a light, camera, or image. Common annotation objects are objects extracted from the image processing pipeline and stored in-memory or in a data store. Annotation objects can also be drawn into the N-dimensional space by the user using drawing tools from the user interface. In a particular implementation, the virtual N-dimensional space exists within a browser based HTML5 canvas object. WebGL, three.js, and shaders are used to instantiate and render light objects, camera objects, images, and annotation objects. Hardware acceleration is leveraged when available. The visualization service provides or implements methods for constructing queries to request images from the image service as a response. Upon receiving images, in a batch or serial format, the service renders the images for display. Constructed queries search for the images by metadata parameters (e.g. experimenter, cell type, etc.) or spatial coordinates. Queries may include a zoom level parameter. Queries are constructed and sent to the image service whenever a camera is initialized or a camera's location changes. Volume rendering techniques may be applied to image stacks to display a 2D projection of the 3D dataset. Common volume rendering techniques include volume ray casting where for a given pixel, a ray is projected through a stack of images or until a specified depth based on the camera's position. Voxels are sampled directly from the raw images if the ray lies along an image. Otherwise, the value is interpolated (a variety of interpolation methods may be used). Next, each sampled value is processed by a shader to determine opacity and color. Finally, the sampled values are composed to determine a final color and opacity for the pixel. Common volume rendering techniques include splatting where an alpha map is applied to the image stack so the highest images are the most transparent. Common volume rendering techniques include shear warp. Common volume rendering techniques include texture-based volume rendering where images can be applied as texture on to objects in the N-dimensional space. Volume stitching may be applied after an initial image stack is retrieved. Volume stitching queries the image service for image stacks proximal to the initial volume in absolute or relative coordinates. After applying a volume rendering technique to each retrieved image stack, each volume is displayed in its correct relative position. The spatial data is viewable from different zoom levels. The zoom level determines which images to query and receive from the image service. A mipmap for each image is created and stored by the image service. Mipmaps can either be dynamically generated when needed or precomputed and stored. The visualization service provides or implements methods for rendering pixels, receiving images from the image server, querying the image server in response to user input, rendering geometric shapes, rendering colors, textures, transparency, 3D layers, etc. The visualization service provides or implements methods for displaying the Graphical User Interface.

### Developer Interface

The SMDS includes a developer interface where particular methods from each service are exposed in a rate-limited, public API. This allows other developers to build plugins that can integrate deeply with the spatial data system.

### User Interface

The user interface is an aesthetic and intuitive interface where the user can interact with the many services. Web elements are constructed with HTML. CSS is used to style web elements, and Javascript dynamically changes content on the web page and in the canvas. The user can login to their account to view data they have uploaded, view public data sets, or choose to upload a new dataset for visualization and analysis. Once a dataset is chosen, the visualization service initializes an N-dimensional space and draws the space to fill the canvas object. The service will query, receive, process, and load images nearby the user's current location in space determined by the position of the camera. Datasets can be searched for in file browser. Images can be queried, received, processed, and loaded into the canvas until they are no longer in the field of view. The user can interact with the canvas with their mouse input. The user can explore the 3D space by panning the camera in the Θ (theta) or Φ (phi) direction, moving the camera, or changing the zoom level. An example mouse interface would be: clicking and dragging will pan the field of view in the direction of the mouse; right clicking and dragging will move the camera's position in the direction of the mouse; scrolling will change the zoom level; scrolling back will zoom out; scrolling forward will zoom in. Every time the field of view changes, the visualization service will react to the change by querying, receiving, processing, and loading the correct set of images to fill the canvas. The user can interact with objects in the N-dimensional space. Clicking an object will bring up an interface, such as an informative sidebar, that will display known information object found in the image processing step. Clicking an object will also select it. The selected object will be distinguished from other objects. The user can search for objects, object groups, or images with a search bar. The search can select an object, group of objects, or images in the current field of view or through the entire experiment data or across the entire database. Summary statistics can be displayed from groups of selected objects when available. Analysis tools and spatial data operations can be applied to selected objects or images. These operations are available through buttons or links that pass necessary information to the underlying analysis service or spatial data service. The service's response will be applied to the current field of view for the user to see. In general, the user interface for molecular data services provides visualization of databases, etc., searching databases, building cross-database queries, a visual interface, and a text interface.

### EXAMPLE II

### General Operation of an Exemplary Sequencing Device

An exemplary sequencing device implements a method of analyzing a plurality of nucleic acids within a three dimensional polymerized matrix including amplifying the plurality of nucleic acids to produce amplicons within the matrix, covalently bonding the amplicons to the matrix, sequencing the plurality of amplicons using an optical sequencing method where the plurality of amplicons are labeled with a detectable label, and volumetrically imaging the plurality of amplicons to produce three dimensional imaging data of the plurality of amplicons wherein light intensity data is processed into a three-dimensional volumetric image. For example, the plurality of nucleic acids may be contained within a biological sample and the matrix-forming material is introduced into the biological sample. The plurality of nucleic acids may be contained within a cell and the matrix-forming material is introduced into the cell. For example, the plurality of nucleic acids may be contained within a tissue sample and the matrix-forming material is introduced into the tissue sample. For example, the three dimensional imaging data identifies the relative position of the plurality of amplicons within the cell. For example, the plurality of amplicons may be sequenced using fluorescence in situ sequencing. For example, the plurality of nucleic acids are volumetrically imaged using one or more of 3D structured illumination, selective planar illumination microscopy, light sheet microscopy, emission manipulation, volumetric imaging using pinhole confocal microscopy, volumetric imaging using aperture correlation confocal microscopy, volumetric imaging using volumetric reconstruction from slices, volumetric imaging using deconvolution microscopy, volumetric imaging using aberration-corrected multifocus microscopy, volumetric imaging using digital holographic microscopy.

An automated sequencing and volumetric imaging device includes a multi axis stage or positioning system including a sample holder for a three dimensional nucleic acid containing matrix, a heating or cooling apparatus operationally connected to the stage, whereby the heating or cooling apparatus is programmable for time and temperature useful with thermo-cycling for amplification and sequencing, a fluidics dispenser positioned to dispense one or more reagents into the sample holder wherein the fluidics dispenser is in fluid communication with one or more reservoirs for containing one or more reagents, whereby the fluidics dispenser is programmable for dispensing programmed volumes of liquid reagents to the sample holder, a pump operationally connected to the fluidics dispenser whereby the pump forces or withdraws one or more reagents from the one or more reservoirs through the fluidics dispenser, an optical assembly including one or more optical axis, one or more detectors positioned in light receiving communication with the sample holder, whereby the one or more detectors receives light intensity signals which processed into a three-dimensional volumetric image of the nucleic acid sample, and one or more microprocessors with software for automating and controlling introduction of reagents into the sample holder, thermocycling of the sample holder, and image detection and acquisition.

### EXAMPLE III

### Computational Method for Processing Raw Image Data from a Sequencing Device

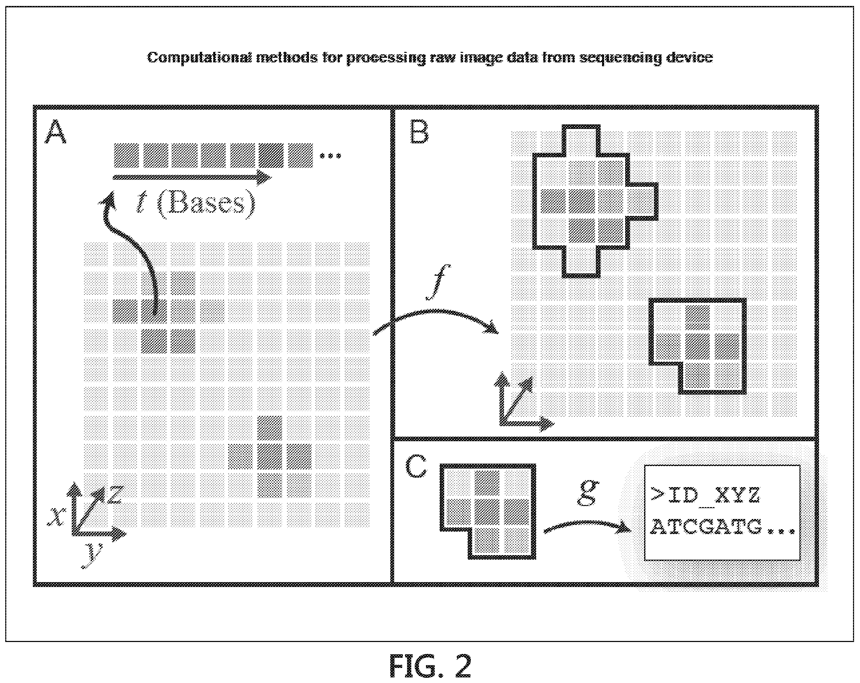

A software functionality implemented by the analysis service may include processing of sequencing image data. Block A of FIG. 2 depicts multi-dimensional sequencing image data, consisting of 3D XYZ pixel values and T pixel values over multiple sequencing bases. Block B of FIG. 2 depicts some function f that clusters pixels to identify sequencing amplicons, such as by using connected component algorithm in "sequence space" or other strategies. Block C of FIG. 2 depicts some function g that generates a sequencing read with spatial coordinates, read, and quality values; e.g. using pixel voting; weighting by quality, distance to centroid, other metrics; or goal-based strategies to maximize alignment quality.

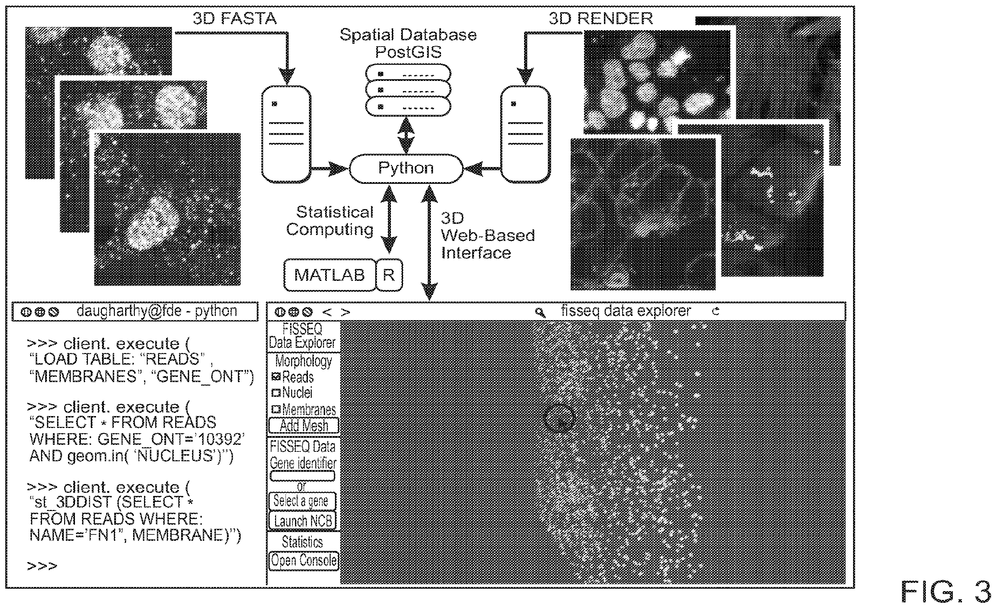

FIG. 3 depicts a system for processing raw image data from a sequencing device. Measurement of genetic elements in 3D space may create volumetric data. Sequencing data is converted into a 3D FASTA, and aligned to the reference to generate a 3D SAM/BAM file. But in the same cells, co-registered measurements can be taken such as nuclear stains, membrane stains, antibodies, and DNA FISH to measure copy-number variation and gene fusions. These information sources can be digitized and represented in the same coordinate space as the sequencing data using a spatial database such as postGIS. This provides a platform from which 3D visualization can be rendered, and statistical computing can be conducted such as querying which genes from a certain gene ontology category are found in the nucleus, or calculating the distances between RNA molecules of a certain species and a cell feature, such as the cell membrane.

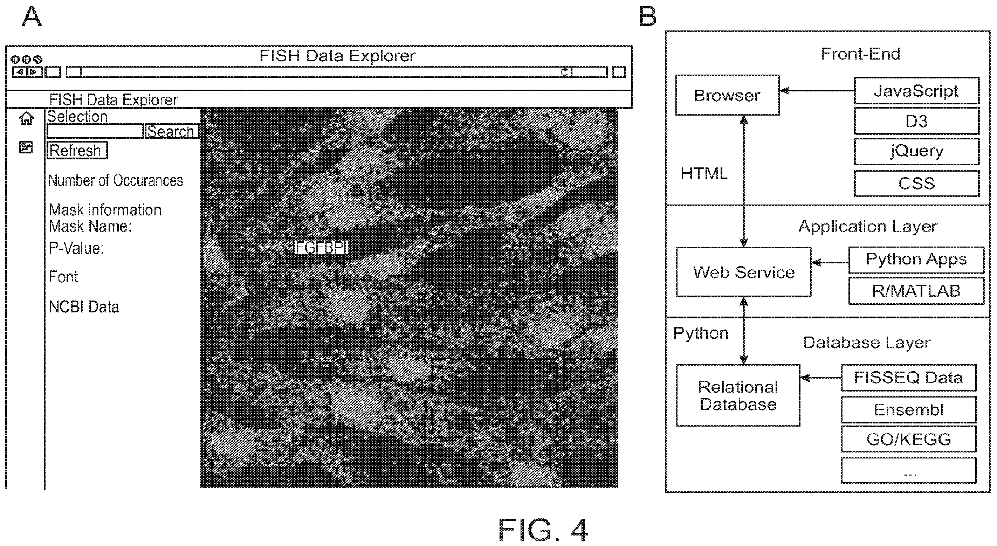

Block A of FIG. 4 depicts a graphical user interface displaying the sequencing read (spatial molecular data), and which allows the user to select certain genes to display and also displays statistical and other molecular data (NCBI data) for each gene or set of genes. Block B of FIG. 4 depicts a front-end browser interface powered by a number of web technologies, and communicating through HTML and other web technologies to the application layer and database layers.

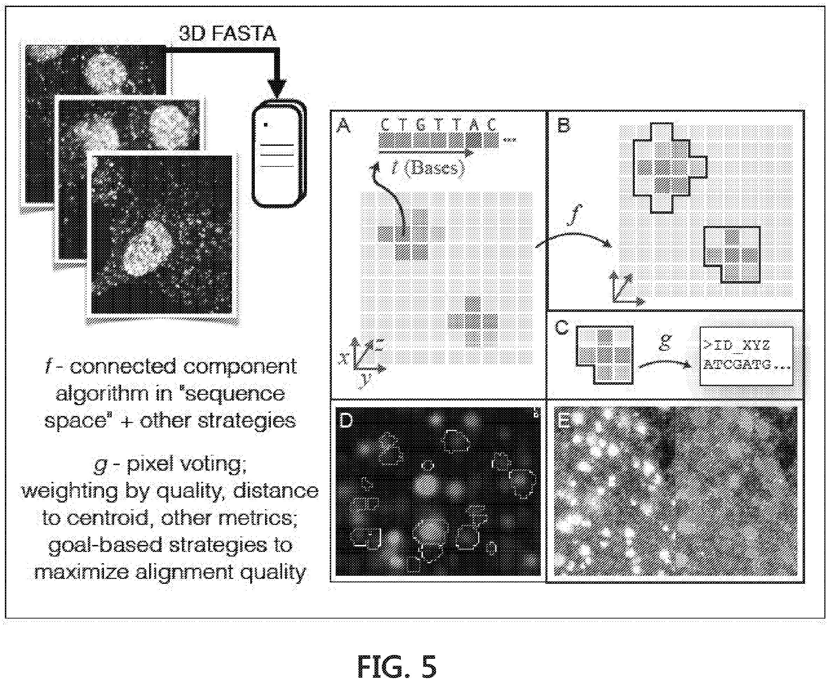

FIG. 5 depicts aspects of processing raw image data from a sequencing device. Block D of FIG. 5 shows an example of sequencing amplicon identification (white outlines). Block E of FIG. 5 shows an example of sequencing amplicon identification (false color on right).

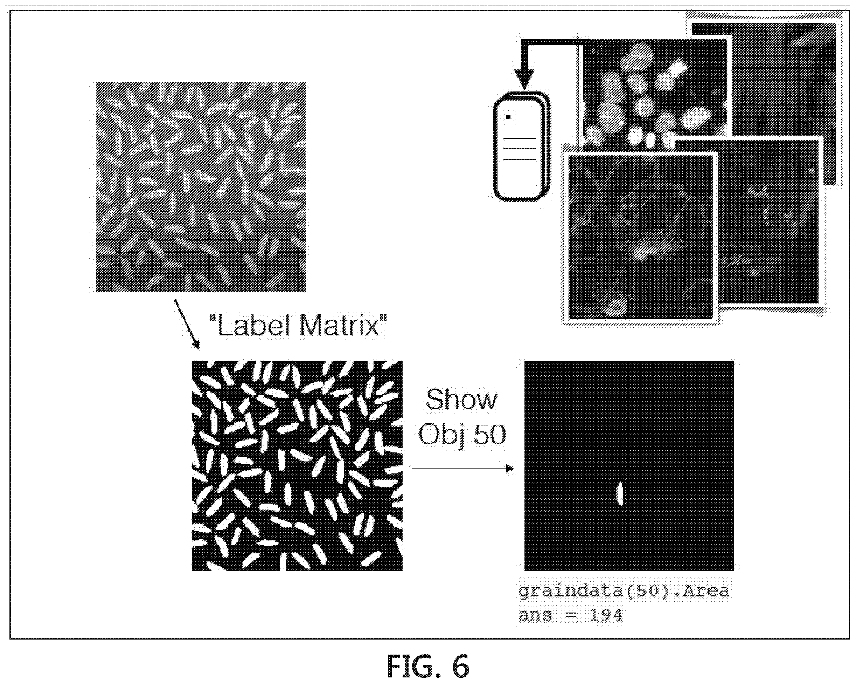

FIG. 6 depicts digitization of image data, such as stains (e.g. nuclear, membrane, antibody, FISH, etc.) and other molecular read-outs. The raw or processed image data is processed into a label matrix where objects are individually identified and features are processed, such as the area of each object.

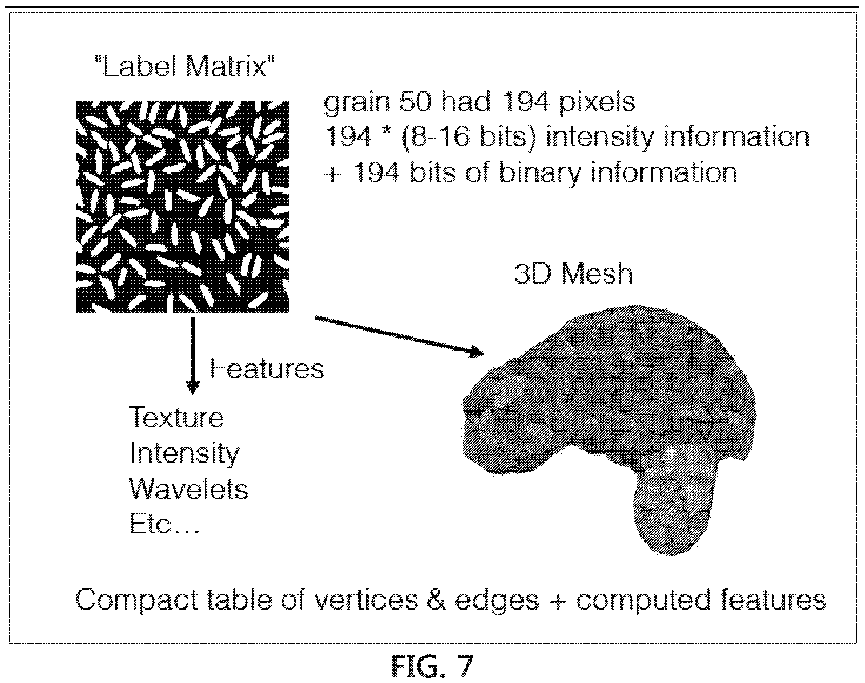

FIG. 7 depicts that the label matrix of objects can be stored as a 3D mesh, e.g. a table of polygons, vertices and edges, or other mesh representations as well as computed features.

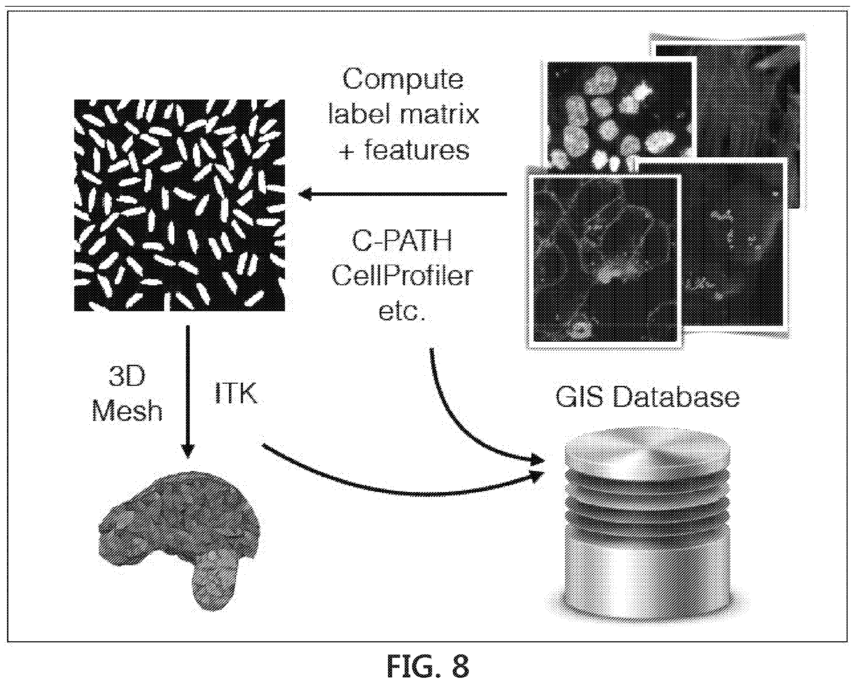

FIG. 8 depicts that the 3D mesh and features are stored in a GIS database for spatial storage, query, and computation.

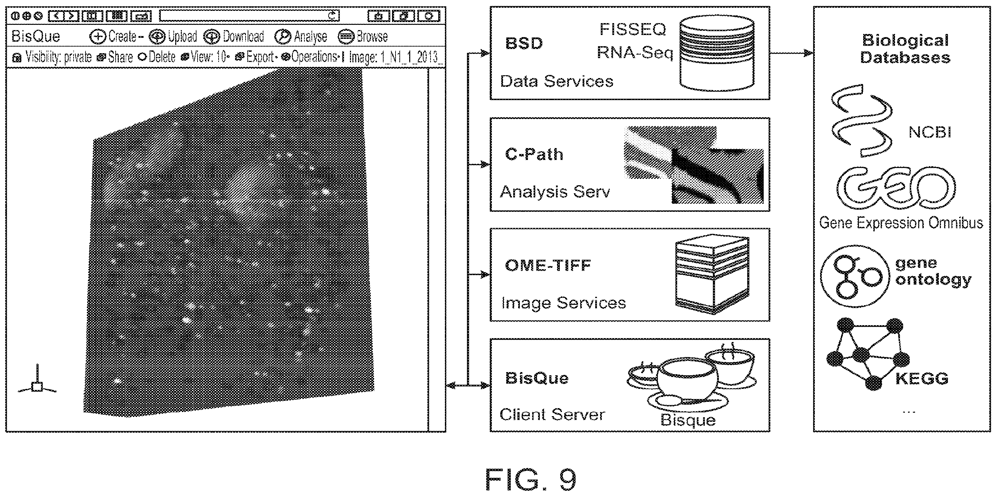

FIG. 9 depicts, in one implementation, that the client service is a BisQue server, the Image service is an OME-TIFF server, and the spatial data service is a Big Spatial Database (BSD) or GIS-based database.

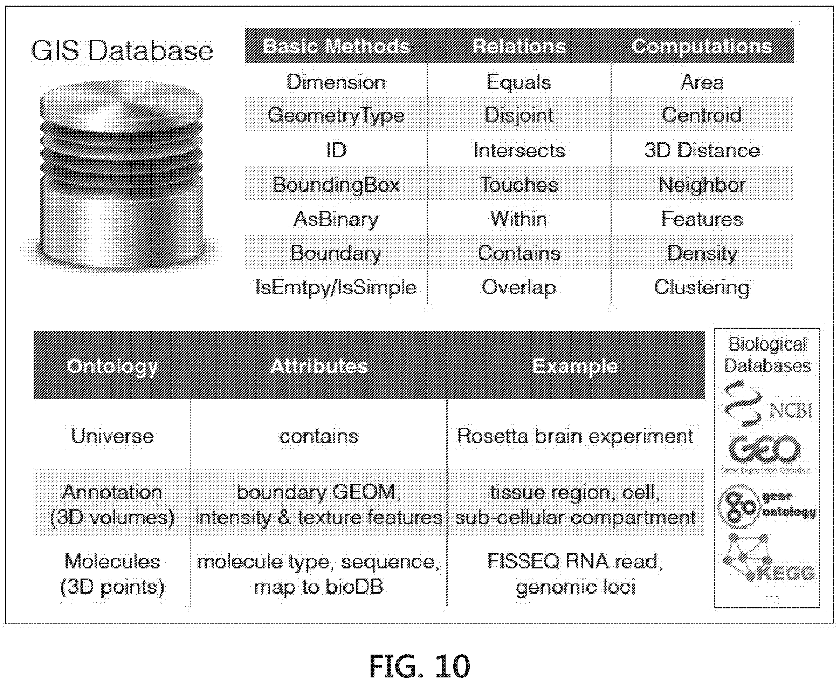

FIG. 10 depicts that the GIS database for spatial data services contains a universe of objects, as well as annotations and molecules. The GIS enables highly efficient spatial computational methods including those in the upper table in FIG. 10.

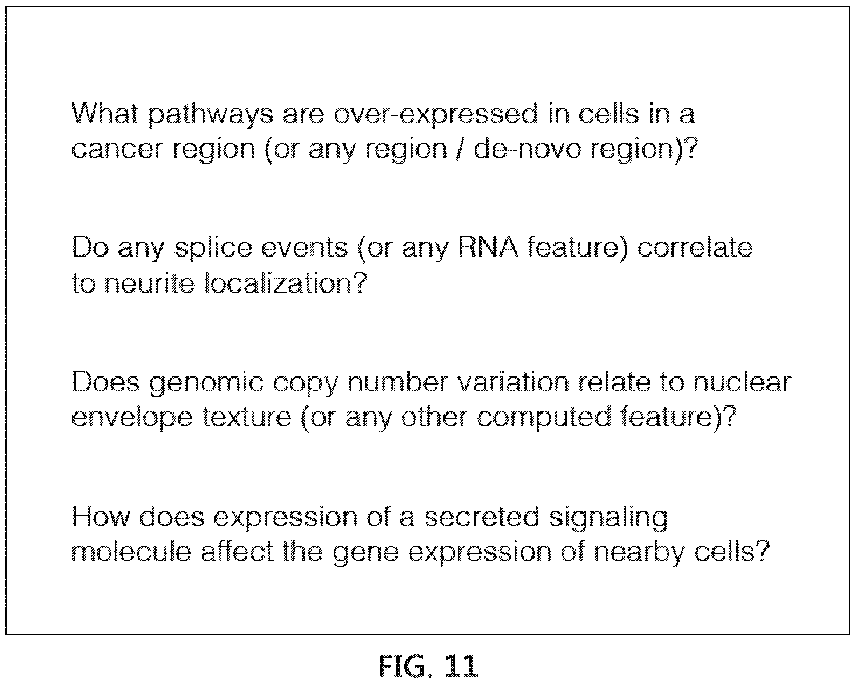

FIG. 11 depicts a software architecture enabling representation of molecular data and computational queries to answer human language questions such as those listed. In certain cases a semantic annotation provides for direct implementation of these types of queries.

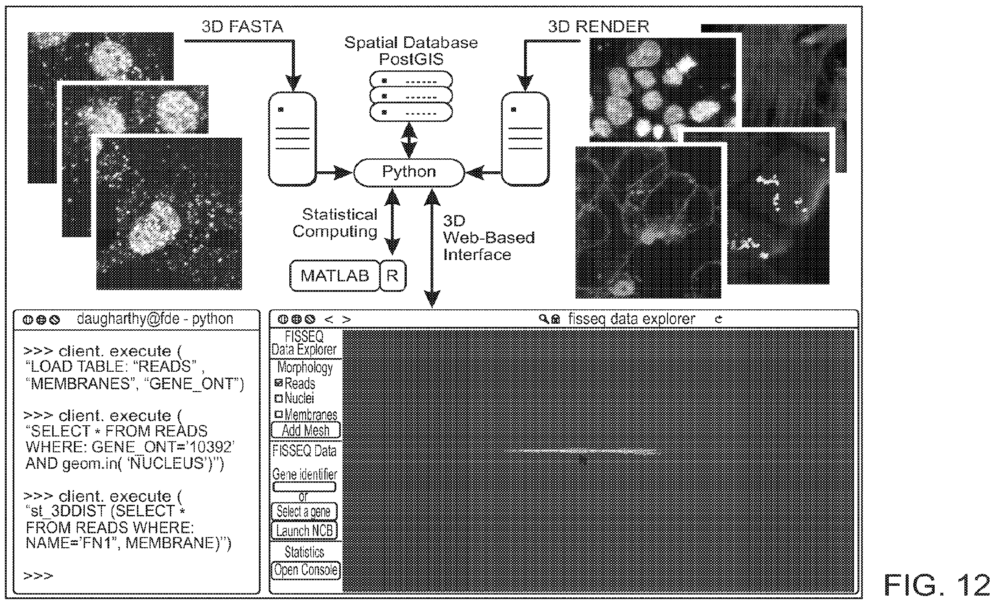

FIG. 12 depicts a system for processing raw image data from a sequencing device. As seen in the bottom right portion of FIG. 12, the user interface may display tools for displaying certain aspects of the data, such as different types of molecular annotations (reads, nuclei, membranes, etc.), search and display of certain genes, or interfaces to NCBI or line-command interfaces. As seen in the bottom left portion of FIG. 12, in command line, structured queries of spatial molecular information may be provided.

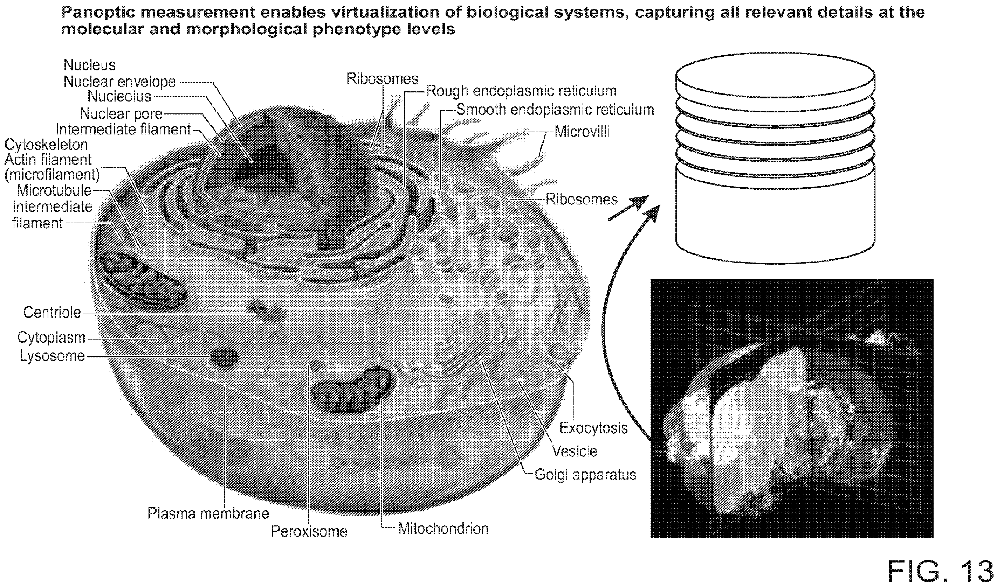

FIG. 13 depicts that the GIS or spatial molecular services enable virtualization of biological systems measured using FISSEQ massively multiplex in situ measurement of RNA, DNA, proteins, and other molecular substrates. This virtualization can represent biological systems across scales such as sub-cellular features (left) or whole-brain features (right).

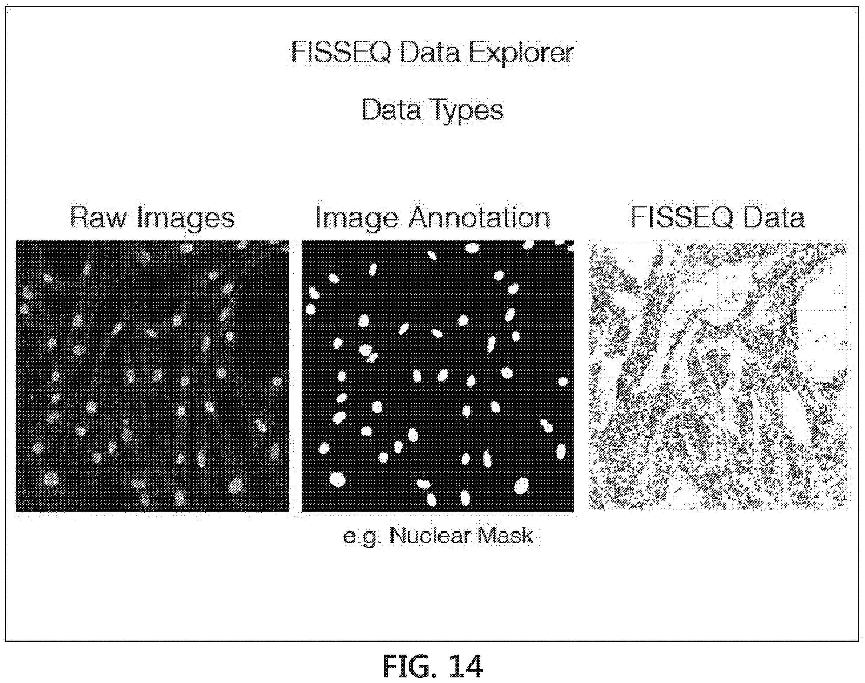

FIG. 14 depicts that basic types of data include raw and processed images (Image Services), image annotations processed from the molecular data or other stains (generated by Analysis Services, images stored by Image Services and 3D mesh representations stored by Spatial Data Services). FISSEQ data is stored by Spatial Data Services but linked to high-dimensional biological annotations via Molecular Data Services and Analysis Services.

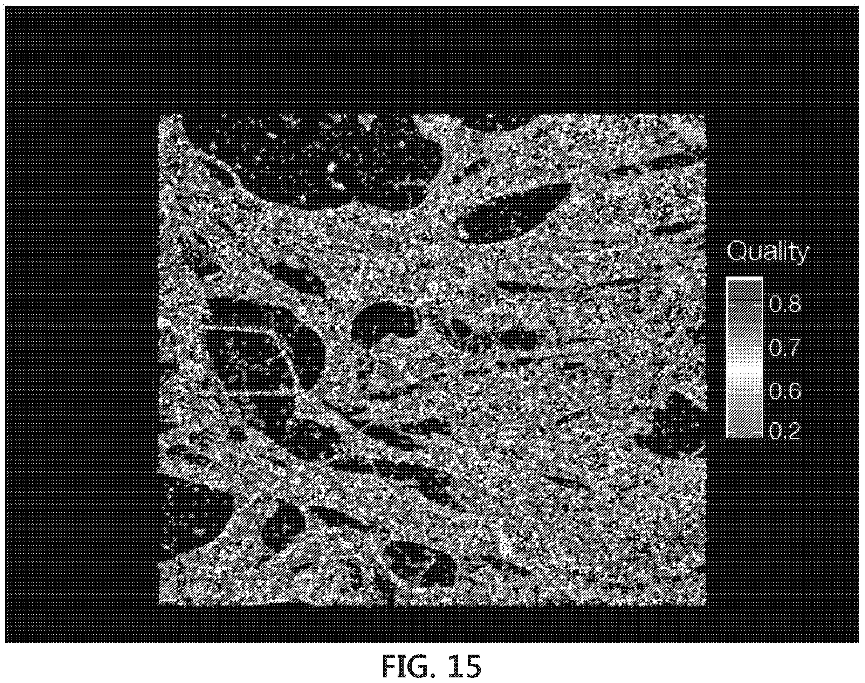

FIG. 15 depicts that many aspects of the data can be visualized, including sequencing read qualities. Abstract visualizations are enabled, such as heatmaps, density plots, etc. These are mediated by Analysis Services and Visualization Services.

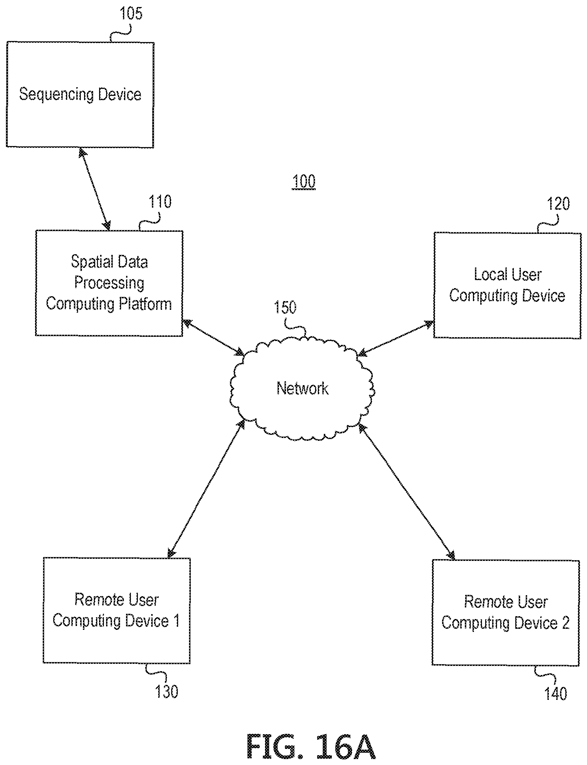

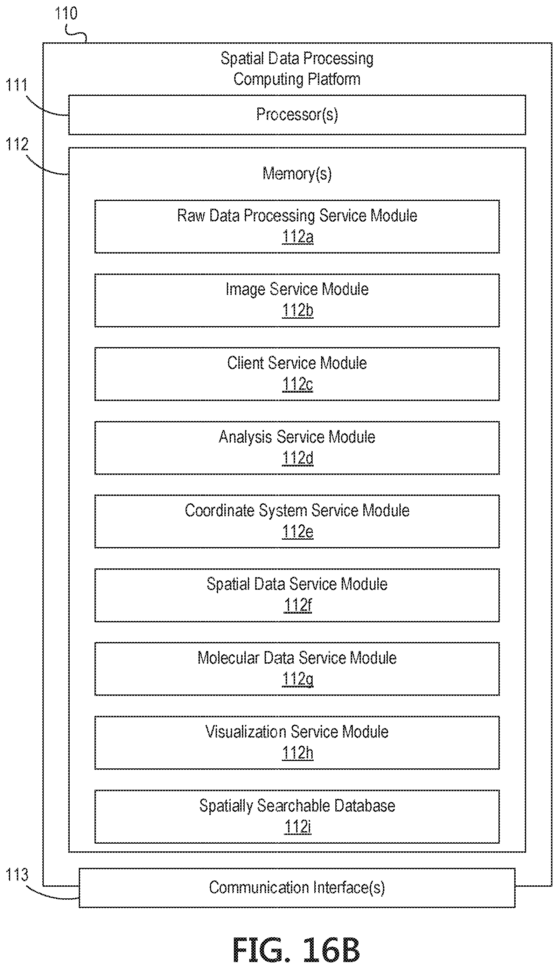

FIGS. 16A and 16B depict an illustrative computing environment for processing spatially related sequence data received from a sequencing device in accordance with one or more example embodiments. Referring to FIG. 16A, computing environment 100 may include one or more sequencing devices. For example, computing environment 100 may include sequencing device 105, which may implement and/or incorporate one or more aspects of the sequencing devices discussed above. For example, sequencing device 105 may be a fluidic sequencing microscope that includes hardware and/or software configured to cause sequencing device 105 to analyze a sample and output raw data to one or more other devices included in computing environment 100. In some instances, sequencing device 105 may receive commands from one or more other devices included in computing environment 100, and such commands may cause sequencing device 105 to perform one or more sequencing operations with respect to a sample and/or output raw data associated with such sequencing operations.

Computing environment 100 also may include one or more computing platforms and other computing devices. For example, computing environment 100 may include spatial data processing computing platform 110, local user computing device 120, remote user computing device 130, and remote user computing device 140. Spatial data processing computing platform 110 may be configured to process spatially related sequence data received from a sequencing device, such as sequencing device 105, as discussed in greater detail below. In addition, spatial data processing computing platform 110 may implement and/or incorporate one or more aspects of the Spatial Molecular Data System discussed above. Spatial data processing computing platform 110 may include one or more computing devices configured to perform one or more of the functions described herein. For example, spatial data processing computing platform 110 may include one or more servers and/or server blades, one or more desktop computers, one or more laptop computers, and/or the like.

Local user computing device 120 may be physically proximate to sequencing device 105 and/or spatial data processing computing platform 110 and may be configured to be used by a local user of sequencing device 105 and/or spatial data processing computing platform 110 in processing spatially related sequence data received from a sequencing device, such as sequencing device 105. For example, local user computing device 120 may be configured to present one or more local user interfaces, receive user input via such user interfaces, and communicate such input to sequencing device 105 and/or spatial data processing computing platform 110 via one or more direct and/or network connections. Remote user computing device 130 and remote user computing device 140 may be physically remote from sequencing device 105 and/or spatial data processing computing platform 110 and may be configured to be used by one or more remote users of sequencing device 105 and/or spatial data processing computing platform 110 in processing spatially related sequence data received from a sequencing device, such as sequencing device 105. For example, remote user computing device 130 and remote user computing device 140 may be configured to present one or more remote user interfaces (e.g., web interfaces, interfaces that leverage partially exposed APIs, etc.), receive user input via such user interfaces, and communicate such input to sequencing device 105 and/or spatial data processing computing platform 110 via one or more network connections. In some instances, local user computing device 120, remote user computing device 130, and remote user computing device 140 may be and/or include desktop computers, laptop computers, tablet computers, smart phones, and/or the like, and may include one or more processors, memories, communication interfaces, storage devices, and/or other components.

Computing environment 100 also may include one or more networks, such as network 150, which may interconnect one or more of spatial data processing computing platform 110, local user computing device 120, remote user computing device 130, and remote user computing device 140. Network 150 may include one or more sub-networks (e.g., local area networks (LANs), wide area networks (WANs), virtual private networks (VPNs), or the like).

Referring to FIG. 16B, spatial data processing computing platform 110 may include one or more processors 111, memory 112, and communication interface 113. In some instances, spatial data processing computing platform 110 also may include an input/output interface that includes a keyboard, mouse, display screen, speaker, microphone, camera, and/or other components. A data bus may interconnect the one or more processors 111, memory 112, communication interface 113, and input/output interface. Communication interface 113 may be a network interface configured to support communication between spatial data processing computing platform 110 and one or more networks (e.g., network 150). Memory 112 may include one or more program modules having instructions that when executed by processor(s) 111 cause spatial data processing computing platform 110 to perform one or more functions described herein and/or one or more databases that may store and/or otherwise maintain information which may be used by such program modules and/or processor(s) 111. In some instances, the one or more program modules and/or databases may be stored by and/or maintained in different memory units of spatial data processing computing platform 110 and/or by different computing devices that may form and/or otherwise make up spatial data processing computing platform 110.

For example, memory 112 may have, store, and/or include one or more modules that may have instructions that direct and/or cause spatial data processing computing platform 110 to implement and/or provide one or more of the services discussed above, such as a raw data processing service module 112a, an image service module 112b, a client service module 112c, an analysis service module 112d, a coordinate system service module 112e, a spatial data service module 112f, a molecular data service module 112g, and a visualization service module 112h. In addition, memory 112 may have, store, and/or include one or more databases, such as spatially searchable database 112i, that may be used by spatial data processing computing platform 110 in processing spatially related sequence data received from a sequencing device, as illustrated in greater detail below. As also illustrated below, in some instances, spatial data processing computing platform 110 may present one or more user interfaces and/or receive user input via a locally-connected display screen (which may, e.g., be a touch-sensitive display screen and/or be included in an input/output interface of spatial data processing computing platform 110) or via a locally-connected computer, such as local user computing device 120, while in other instances, spatial data processing computing platform 110 may present one or user interfaces and/or receive user input via a remote, network-connected computer, such as remote user computing device 130 and/or remote user computing device 140.

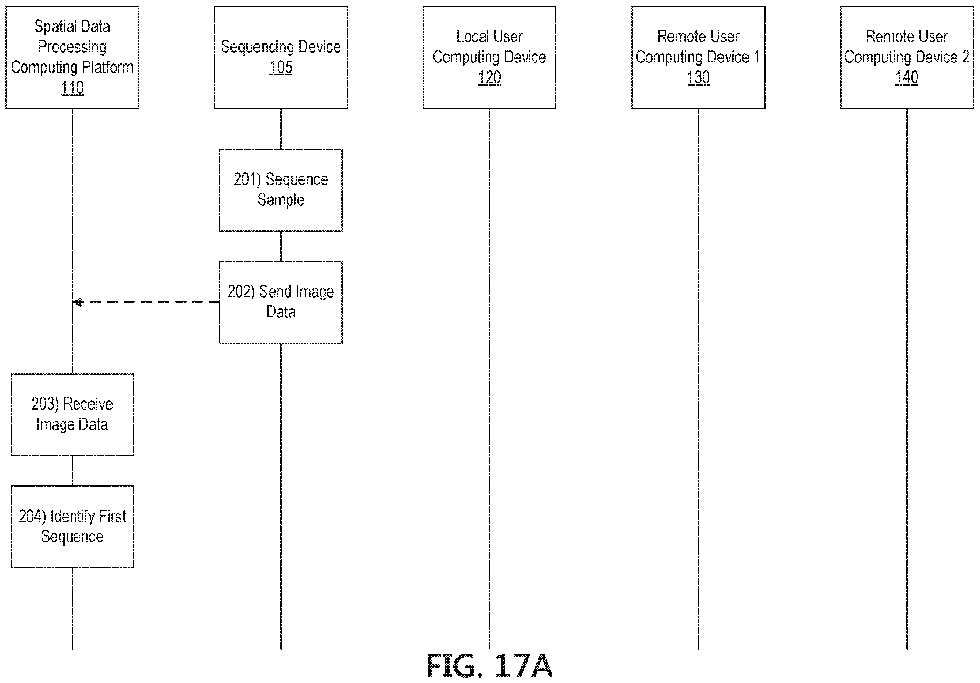

FIGS. 17A-17E depict an illustrative event sequence for processing spatially related sequence data received from a sequencing device in accordance with one or more example embodiments. Referring to FIG. 17A, at step 201, sequencing device 105 may sequence a sample. For example, in sequencing the sample at step 201, sequencing device 105 may execute a protocol of sequencing and imaging of a three dimensional nucleic acid containing matrix which is contained within a suitable vessel or stage. In addition, in sequencing the sample at step 201, sequencing device 105 may obtain two-dimensional image data and/or three-dimensional image data associated with the sample. Additionally or alternatively, in sequencing the sample at step 201, sequencing device 105 may perform one or more other operations, including one or more of the operations of the sequencing device discussed above with respect to FIG. 1.

At step 202, sequencing device 105 may send image data and/or other raw data obtained from and/or otherwise associated with the sequenced sample to spatial data processing computing platform 110 for processing. For example, in sending image data and/or other raw data obtained from and/or otherwise associated with the sequenced sample to spatial data processing computing platform 110 at step 202, sequencing device 105 may send a raw data stream to spatial data processing computing platform 110. The raw data stream (which may, e.g., be sent by sequencing device 105 to spatial data processing computing platform 110) may, for instance, include pixel values associated with the sample and/or metadata associated with the image data associated with the sample, as discussed above with respect to FIG. 1.

At step 203, spatial data processing computing platform 110 may receive the image data and/or other raw data from sequencing device 105. For example, at step 203, spatial data processing computing platform 110 may receive, from a sequencing device (e.g., sequencing device 105), image data associated with a sample. In some instances, in receiving the image data associated with the sample, spatial data processing computing platform 110 may receive a raw data stream that includes image data associated with the sample, as discussed above.

In some instances, the image data received by spatial data processing computing platform 110 from sequencing device 105 at step 203 may include image metadata. Such image metadata may, for example, include timing information indicating time(s) at which the image data was obtained from the sample, position information indicating position(s) at which the image data was obtained from the sample, and/or other information. For instance, the image metadata may additionally or alternatively include information identifying who performed the experiment, information identifying who prepared the sample, information specifying a patient identifier or patient identifier code associated with the sample, information identifying environmental conditions when the image data was obtained from the sample (e.g., temperature, humidity, etc.), and/or information identifying other types of medical and/or biological annotations that may accompany the image data.

In some embodiments, the image data received from the sequencing device may include spatial information, temporal information, and color information associated with the sample. For example, the image data received (which may, e.g., be received by spatial data processing computing platform 110 at step 203) from the sequencing device (e.g., sequencing device 105) may include spatial information, temporal information, and color information associated with the sample. The color information associated with the sample may include wavelength information, as color corresponds to a wavelength of electromagnetic radiation. Although sequencing device 105 may typically use wavelengths in the visual spectrum when sequencing a sample, sequencing device 105 may, in some instances, use wavelengths that are not in the visual spectrum when sequencing a particular sample.

In some embodiments, the sequencing device may be configured to sequence the sample using fluorescent in situ sequencing (FISSEQ). For example, sequencing device 105 may be configured to sequence the sample using FISSEQ, as discussed above, and in receiving the image data associated with the sample, spatial data processing computing platform 110 may receive, from sequencing device 105, image data that was obtained by sequencing device 105 using FISSEQ.

At step 204, spatial data processing computing platform 110 may identify a first sequence in the sample. For example, at step 204, spatial data processing computing platform 110 may identify, based on the image data received from the sequencing device (e.g., sequencing device 105), a first sequence located at first spatial coordinates. In identifying the first sequence located at the first spatial coordinates, spatial data processing computing platform 110 may identify a set of temporally-ordered and/or spatially-ordered signals, which may represent and/or make up the identified sequence (e.g., using one or more functions that cluster pixels of the multi-dimensional sequencing image data to identify sequencing amplicons and/or generate a sequencing read with spatial coordinates, read, and quality values, as discussed above and as illustrated in FIG. 2). For example, in identifying the first sequence located at the first spatial coordinates, spatial data processing computing platform 110 may identify the presence of a specific nucleic acid sequence at the first spatial coordinates, such as a particular DNA sequence, RNA sequence, or the like. In some instances, the first spatial coordinates (e.g., at which spatial data processing computing platform 110 may identify the first sequence) may be point coordinates. In many instances, the first spatial coordinates (e.g., at which spatial data processing computing platform 110 may identify the first sequence) may include sets of coordinates that define a geometry and/or specify a single object corresponding to the first sequence.

In some instances, in addition to identifying the first sequence located at the first spatial coordinates, spatial data processing computing platform 110 also may extract other features from the image data, such as texture information, intensity information, and/or other information, as discussed above and as illustrated in FIG. 7, for example. These other features may be linked to the geometry and/or object associated with the sequence, and these features may be stored in a spatially searchable database along with object data defining the geometry and/or object associated with the sequence. In addition, these other features may represent and/or define annotations of the object associated with the sequence that may be derived (e.g., by spatial data processing computing platform 110) from the image data, rather than being annotations that may be derived from a look-up table. For instance, some annotations of the object associated with the sequence may be derived by spatial data processing computing platform 110 from the image data, such as texture and intensity, while other annotations of the object associated with the sequence may be derived by spatial data processing computing platform 110 from a look-up table, such as gene name.

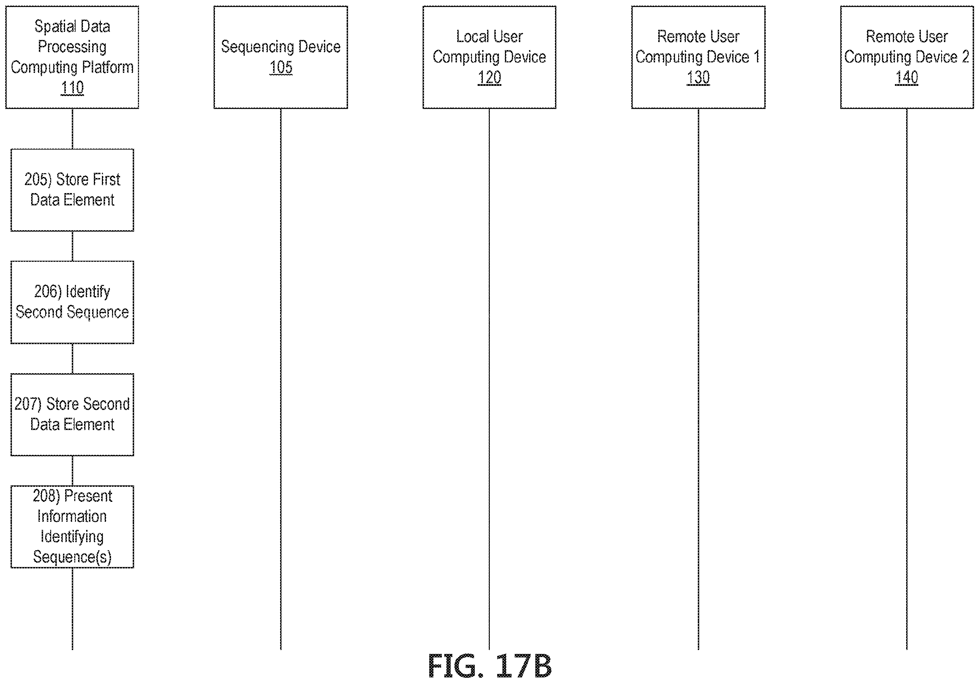

Referring to FIG. 17B, at step 205, spatial data processing computing platform 110 may store a first data element (e.g., based on identifying the first sequence in the sample). For example, at step 205, spatial data processing computing platform 110 may store, in a spatially searchable database (e.g., spatially searchable database 112i), a first data element comprising the first spatial coordinates and a first identifier corresponding to the first sequence to spatially relate the first sequence to other sequences present in the sample. In storing the first data element comprising the first spatial coordinates and the first identifier, spatial data processing computing platform 110 may, for instance, store a data element similar to the example data element illustrated in Block C of FIG. 2. For example, and as seen in FIG. 2, ">ID_XYZ" may be a data element that corresponds to an identified sequence. The data element may have an identifier component that identifies the sequence (e.g., "ID") and three-dimensional spatial coordinates that identify the location of the sequence (e.g., "XYZ"). The identifier component may be a key in a database table stored in spatially searchable database 112i and/or an informatic identifier that identifies the sequence. In addition, and as discussed above, creating and/or utilizing such a data element may be referred to as implementing "3D FASTA," insofar as expands on FASTA to identify the presence of a particular sequence at a particular three-dimensional location in a sequenced sample.

In some embodiments, the spatially searchable database may be maintained in a geographic information system (GIS) format. For example, the spatially searchable database (e.g., spatially searchable database 112i, in which spatial data processing computing platform 110 may store the first data element at step 205) may be maintained in a GIS format, which may provide for spatial queries of the data and spatial computations such as 3D distance and containment (within), as discussed above.

At step 206, spatial data processing computing platform 110 may identify a second sequence in the sample. For example, at step 206, spatial data processing computing platform 110 may identify, based on the image data received from the sequencing device (e.g., sequencing device 105), a second sequence located at second spatial coordinates. Spatial data processing computing platform 110 may, for instance, identify the second sequence similar to how spatial data processing computing platform 110 may identify the first sequence, and the second sequence may correspond to and/or represent a different object located at different spatial coordinates than the first sequence.

At step 207, spatial data processing computing platform 110 may store a second data element (e.g., based on identifying the second sequence in the sample). For example, at step 207, spatial data processing computing platform 110 may store, in the spatially searchable database (e.g., spatially searchable database 112i), a second data element comprising the second spatial coordinates and a second identifier corresponding to the second sequence to spatially relate the second sequence to the other sequences present in the sample. Similar to storing the first data element, in storing the second data element comprising the second spatial coordinates and the second identifier, spatial data processing computing platform 110 may, for instance, store a data element similar to the example data element illustrated in Block C of FIG. 2. For example, the data element may have an identifier component that identifies the sequence (e.g., "ID") and three-dimensional spatial coordinates that identify the location of the sequence (e.g., "XYZ").

At step 208, spatial data processing computing platform 110 may present information identifying the first sequence and/or the second sequence. For example, at step 208, spatial data processing computing platform 110 may present, on a display device, information identifying a presence of the first sequence at the first spatial coordinates. In some instances, the display device (e.g., on which spatial data processing computing platform 110 may present the information identifying the presence of the first sequence at the first spatial coordinates and/or the presence of the second sequence at the second spatial coordinates) may be a display device that is included in and/or directly connected to an input/output interface of spatial data processing computing platform 110. In some instances, the display device (e.g., on which spatial data processing computing platform 110 may present the information identifying the presence of the first sequence at the first spatial coordinates and/or the presence of the second sequence at the second spatial coordinates) may be a display device may be a display device that is connected to and/or included in another computing device, such as local user computing device 120, remote user computing device 130, and/or remote user computing device 140.

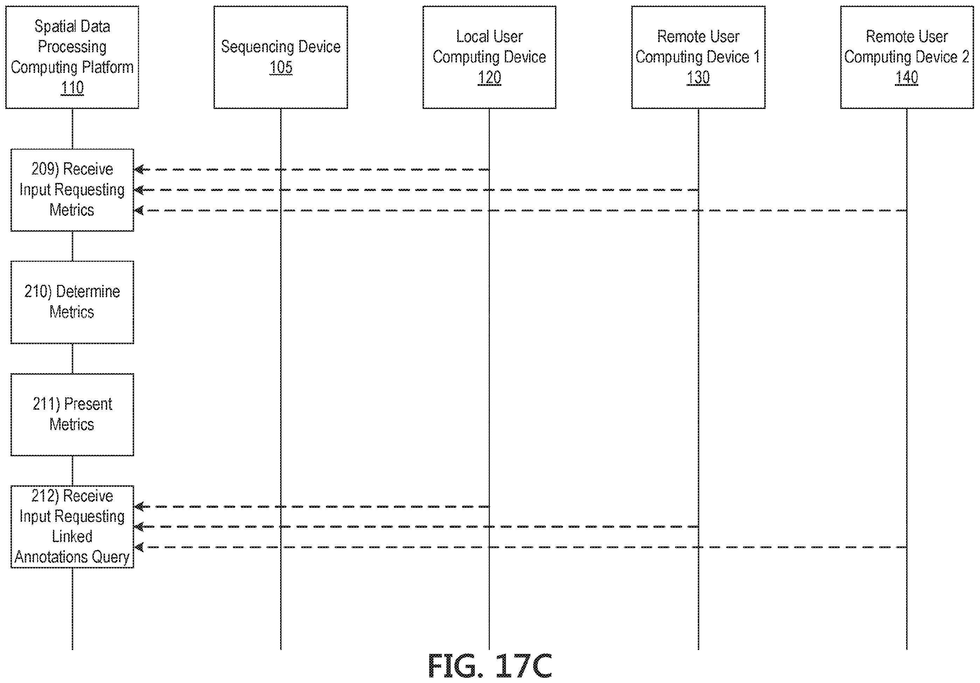

Referring to FIG. 17C, at step 209, spatial data processing computing platform 110 may receive input requesting metrics associated with the sample. Such input may, for example, be received from a user of spatial data processing computing platform 110 interacting with spatial data processing computing platform 110 via a display device and/or other input/output interface connected to spatial data processing computing platform 110, or such input may be received from another computing device (e.g., local user computing device 120, remote user computing device 130, remote user computing device 140) via a communication interface (e.g., communication interface 113).

At step 210, spatial data processing computing platform 110 may determine one or more metrics associated with the sample (e.g., based on the input requesting the metrics associated with the sample). For example, at step 210, spatial data processing computing platform 110 may determine one or more metrics associated with the first sequence at the first spatial coordinates.

In determining the one or more metrics associated with the first sequence at the first spatial coordinates, spatial data processing computing platform 110 may, for example, calculate and/or otherwise determine an object size associated with the first sequence, one or more texture values associated with the first sequence, one or more intensity values associated with the first sequence, one or more sequencing quality values associated with the first sequence, and/or other metrics based on the features of the object in the image data. In some instances, spatial data processing computing platform 110 may compute and/or otherwise determine such metrics purely with respect to the object associated with the first sequence, while in other instances, spatial data processing computing platform 110 may compute and/or otherwise determine such metrics in a manner that requires joint computations involving more than one object. For example, some metrics (which may, e.g., be computed and/or otherwise determined by spatial data processing computing platform 110) may be determined based solely on the object being analyzed, such as size, texture, centroid, regularity (which may, e.g., indicate how round or jagged the object is), and/or inertial moment. Other metrics (which may, e.g., be computed and/or otherwise determined by spatial data processing computing platform 110) may be determined relative to other objects in addition to the particular object being analyzed, such as sequencing quality. For instance, in determining the sequencing quality of a particular object, spatial data processing computing platform 110 may determine a distribution of objects in the sample and/or in the database, and then spatial data processing computing platform 110 may score individual objects relative to that distribution. In some instances, this determination may involve referencing a look-up table and/or determining one or more Phred quality scores, which are quality metrics that can be used with sequencing data.

At step 211, spatial data processing computing platform 110 may present the one or more metrics associated with the sample. For example, at step 211, spatial data processing computing platform 110 may present, on a display device, information identifying the one or more metrics associated with the first sequence at the first spatial coordinates. For example, spatial data processing computing platform 110 may present such metrics via a display device and/or other input/output interface connected to spatial data processing computing platform 110, and/or spatial data processing computing platform 110 may present such metrics by directing and/or causing another computing device (e.g., local user computing device 120, remote user computing device 130, remote user computing device 140) to present such metrics.

At step 212, spatial data processing computing platform 110 may receive input requesting a linked annotations query. Such input may, for example, be received from a user of spatial data processing computing platform 110 interacting with spatial data processing computing platform 110 via a display device and/or other input/output interface connected to spatial data processing computing platform 110, or such input may be received from another computing device (e.g., local user computing device 120, remote user computing device 130, remote user computing device 140) via a communication interface (e.g., communication interface 113).

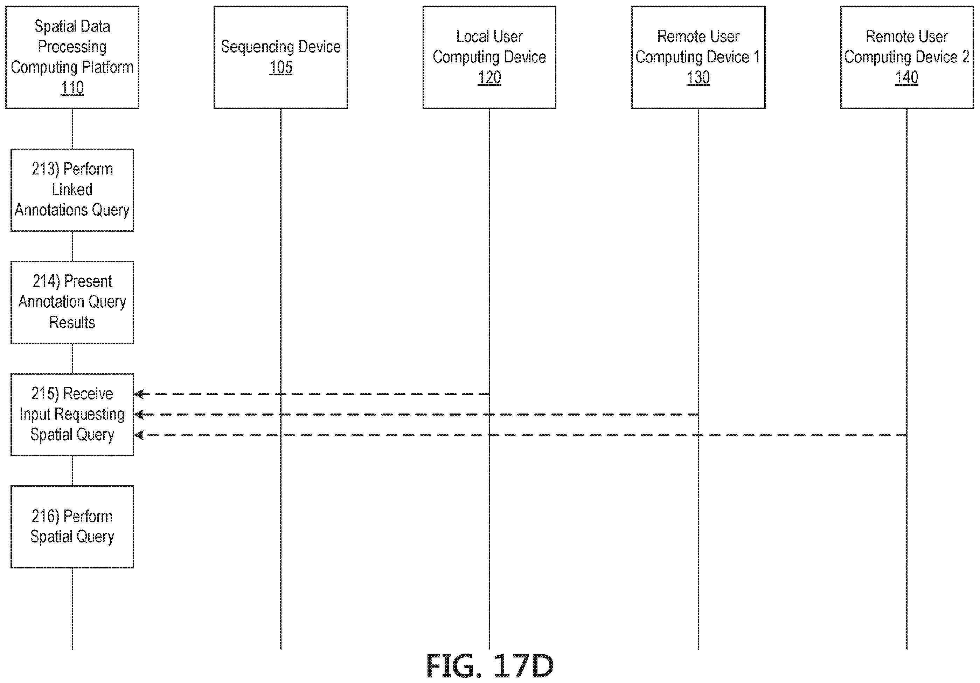

Referring to FIG. 17D, at step 213, spatial data processing computing platform 110 may perform a linked annotations query (e.g., based on the input requesting the linked annotations query). For example, at step 213, spatial data processing computing platform 110 may perform a linked annotations query on the spatially searchable database (e.g., spatially searchable database 112i) to obtain annotation query results information. Annotations may be linked to specific sequences by unique identifiers. For instance, a particular unique identifier may correspond to a particular gene name (e.g., FN2). Thus, the annotation query results information may include one or more gene names and/or other annotations obtained by spatial data processing computing platform 110 from spatially searchable database 112i based on a unique identifier associated with the particular sequence(s) being queried.

When performing a linked annotations query, in addition to looking up one or more annotations based on an identifier corresponding to a sequence, spatial data processing computing platform 110 also may use other information, such as user-provided information and/or image metadata, in looking up and/or otherwise obtaining one or more annotations corresponding to a sequence. For example, in performing a linked annotations query, spatial data processing computing platform 110 may receive user-provided species information and/or image metadata indicating that the particular sample being analyzed corresponds to a human species, a mouse species, or another species, and spatial data processing computing platform 110 may select a particular database or look-up table to query based on the species indicated by the user-provided species information and/or the image metadata. In some instances, such image metadata may be generated by sequencing device 105 based on input received by sequencing device 105, and sequencing device 105 may transmit the image metadata to spatial data processing computing platform 110 with the image data, as discussed above. In some instances, annotations may be linked to identifiers using methods that are known in the field of bioinformatics. For example, one or more algorithms may be used to map sequencing reads to the reference database to identify that a particular sequencing read corresponds to a particular gene.

In some instances, one or more annotations may be generated and/or identified by one or more other systems different from spatial data processing computing platform 110. For example, one or more computing devices different from spatial data processing computing platform 110 (e.g., local user computing device 120, remote user computing device 130, and/or remote user computing device 140) may execute software to generate and/or identify one or more annotations for a particular sample (e.g., software to identify one or more mutations in the particular sample). In addition, the one or more computing devices different from spatial data processing computing platform 110 (e.g., local user computing device 120, remote user computing device 130, and/or remote user computing device 140) may store information associated with the one or more generated and/or identified annotations in a database maintained by spatial data processing computing platform 110 (e.g., spatially searchable database 112i). Thus, in querying annotations associated with a particular sample, spatial data processing computing platform 110 may query one or more annotations generated and/or identified by spatial data processing computing platform 110 and/or one or more annotations generated and/or identified by one or more computing devices different from spatial data processing computing platform 110 (e.g., local user computing device 120, remote user computing device 130, and/or remote user computing device 140).

At step 214, spatial data processing computing platform 110 may present annotation query results. For example, at step 214, spatial data processing computing platform 110 may present, on a display device, at least a portion of the annotation query results information. For example, spatial data processing computing platform 110 may present such query results via a display device and/or other input/output interface connected to spatial data processing computing platform 110, and/or spatial data processing computing platform 110 may present such query results by directing and/or causing another computing device (e.g., local user computing device 120, remote user computing device 130, remote user computing device 140) to present such query results.

At step 215, spatial data processing computing platform 110 may receive input requesting a spatial query. Such input may, for example, be received from a user of spatial data processing computing platform 110 interacting with spatial data processing computing platform 110 via a display device and/or other input/output interface connected to spatial data processing computing platform 110, or such input may be received from another computing device (e.g., local user computing device 120, remote user computing device 130, remote user computing device 140) via a communication interface (e.g., communication interface 113). For example, spatial data processing computing platform 110 may receive user input requesting a spatial query, and spatial data processing computing platform 110 may perform a spatial query in response to receiving the user input requesting the spatial query, as illustrated below.

At step 216, spatial data processing computing platform 110 may perform a spatial query (e.g., based on the input requesting the spatial query). For example, spatial data processing computing platform 110 may perform a spatial query on the spatially searchable database (e.g., spatially searchable database 112i) to obtain spatial query results information.

In some embodiments, performing the spatial query on the spatially searchable database may include jointly querying spatial coordinates data and linked annotations data maintained in the spatially searchable database. For example, in performing the spatial query on the spatially searchable database (e.g., spatially searchable database 112i), spatial data processing computing platform 110 may jointly query spatial coordinates data and linked annotations data maintained in the spatially searchable database (e.g., spatially searchable database 112i). For instance, spatial data processing computing platform 110 may perform a query to identify sequences that are located in particular positions (e.g., such as within particular ranges of coordinates, based on the spatial coordinates data) and that have certain annotations (e.g., such as particular gene names, based on the linked annotations data).

For example, in performing the spatial query on the spatially searchable database (e.g., spatially searchable database 112i), spatial data processing computing platform 110 may jointly query spatial coordinates data and linked annotations data maintained in the spatially searchable database to identify instances where two specific molecules are located within a certain distance of each other and/or are otherwise within a defined proximity of each other. As another example, spatial data processing computing platform 110 may jointly query particular tissue regions and particular genes that are involved in certain biological and/or functional pathways. In some instances, in performing such a query, spatial data processing computing platform 110 also may query a KEGG database that maintains information associated with various gene pathways. Additionally or alternatively, spatial data processing computing platform 110 may store information associated with KEGG annotations, and such information may be used by spatial data processing computing platform 110 in querying a particular spatial region of a sample, such as a particular tissue region.

In some embodiments, performing the spatial query on the spatially searchable database may include performing a three-dimensional distance query to identify a three-dimensional distance between the first sequence and another sequence present in the sample. For example, in performing the spatial query on the spatially searchable database (e.g., spatially searchable database 112i), spatial data processing computing platform 110 may perform a three-dimensional distance query to identify a three-dimensional distance between the first sequence and another sequence present in the sample. For instance, spatial data processing computing platform 110 may perform a three-dimensional distance query to calculate the three-dimensional distance between the first sequence and the second sequence (e.g., based on the spatial coordinates data stored in spatially searchable database 112i identifying the locations of the first sequence and the second sequence in the sample).

In some embodiments, performing the spatial query on the spatially searchable database may include performing a containment query to identify a position of the first sequence relative to other features of the sample. For example, in performing the spatial query on the spatially searchable database (e.g., spatially searchable database 112i), spatial data processing computing platform 110 may perform a containment query to identify a position of the first sequence relative to other features of the sample. For instance, spatial data processing computing platform 110 may perform a containment query to determine whether the first sequence is contained by and/or within other features of the sample (e.g., based on the spatial coordinates data stored in spatially searchable database 112i identifying the locations of the first sequence and the other features in the sample).

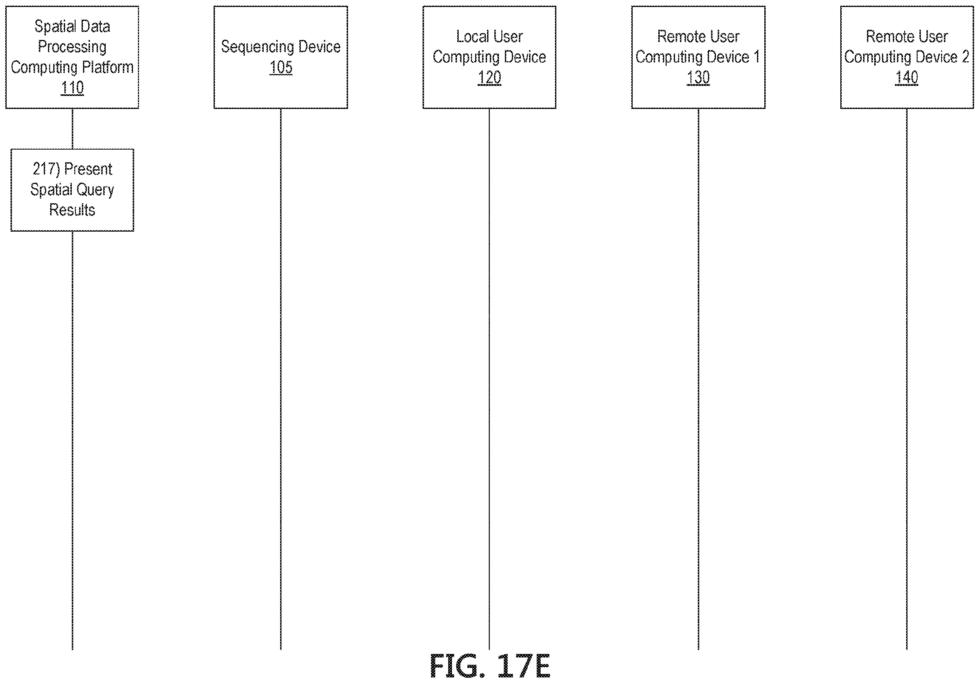

Referring to FIG. 17E, at step 217, spatial data processing computing platform 110 may present spatial query results. For example, at step 217, spatial data processing computing platform 110 may present, on a display device, at least a portion of the spatial query results information. For example, spatial data processing computing platform 110 may present such query results via a display device and/or other input/output interface connected to spatial data processing computing platform 110, and/or spatial data processing computing platform 110 may present such query results by directing and/or causing another computing device (e.g., local user computing device 120, remote user computing device 130, remote user computing device 140) to present such query results.

The steps of the example event sequence discussed above are illustrative and may be performed in a different order than described in some instances. In addition, one or more steps may be optional. In some instances, spatial data processing computing platform 110 may repeat one or more steps, for instance, in identifying and/or storing one or more data elements for one or more other sequences that may be present in the same sample or in a different sample.

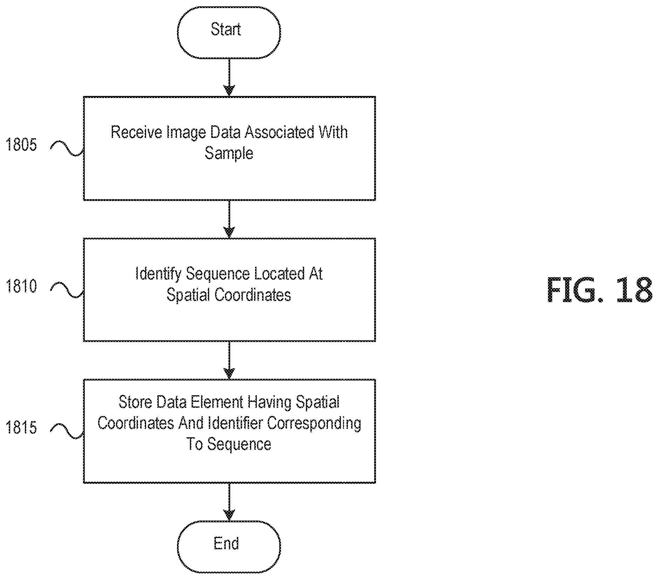

FIG. 18 depicts an illustrative method for processing spatially related sequence data received from a sequencing device in accordance with one or more example embodiments. Referring to FIG. 18, at step 1805, a computer system having at least one processor and memory may receive, from a sequencing device, image data associated with a sample. At step 1810, the computer system may identify, based on the image data received from the sequencing device, a first sequence located at first spatial coordinates. At step 1815, the computer system may store, in a spatially searchable database, a first data element comprising the first spatial coordinates and a first identifier corresponding to the first sequence to spatially relate the first sequence to other sequences present in the sample.

As illustrated above, one or more aspects of the disclosure thus encompass and/or provide the following sample embodiments:
1. A computer implemented system for processing spatial molecular data including: a raw data processing service which receives raw data from a sequencing device; an image service for receiving, storing, and serving images; a coordinate system service that uses one or more numbers, or coordinates, to determine the position of geometric elements on a manifold; a spatial data service for storing, retrieving and processing of spatial data and spatial relationships; a molecular data service for receiving, updating, version tracking, managing and querying relational biological databases; an analysis service for providing access to data analysis packages; a visualization service initializing an N-dimensional space, constructing queries to request images from an image service and rendering the images for display; software APIs and a GUI; a client service; a developer interface; and a user interface.
2. The computer implemented system of embodiment 1 wherein the raw data processing service exchanges data with and is communicatively coupled to the image service and the coordinate system service.
3. The computer implemented system of embodiment 1 wherein the image service exchanges data with and is communicatively coupled to the raw data processing service, the client service, the coordinate system service and the analysis service.
4. The computer implemented system of embodiment 1 wherein the client service exchanges data with and is communicatively coupled to the image service, the visualization service, the software APIs and GUI, the coordinate system service, the spatial data service and the analysis service.
5. The computer implemented system of embodiment 1 wherein the visualization service exchanges data with and is communicatively coupled to client service and the software APIs and GUI.
6. The computer implemented system of embodiment 1 wherein the coordinate system service exchanges data with and is communicatively coupled to image service, the spatial data service, the analysis service, the client service, and the raw data processing service.
7. The computer implemented system of embodiment 1 wherein the software APIs and GUI exchanges data with and is communicatively coupled to the visualization service and the client service.
8. The computer implemented system of embodiment 1 wherein the analysis service exchanges data with and is communicatively coupled to the image service, the client service, the coordinate system, the spatial data service and the molecular data service.
9. The computer implemented system of embodiment 1 wherein the molecular data service exchanges data with and is communicatively coupled to the analysis service.
10. The computer implemented system of embodiment 1 wherein the spatial data service exchanges data with and is communicatively coupled to the client service, the coordinate system, and the analysis service.

One or more aspects of the disclosure may be embodied in computer-usable data or computer-executable instructions, such as in one or more program modules, executed by one or more computers or other devices to perform the operations described herein. Generally, program modules include routines, programs, objects, components, data structures, and the like that perform particular tasks or implement particular abstract data types when executed by one or more processors in a computer or other data processing device. The computer-executable instructions may be stored as computer-readable instructions on a computer-readable medium such as a hard disk, optical disk, removable storage media, solid-state memory, RAM, and the like. The functionality of the program modules may be combined or distributed as desired in various embodiments. In addition, the functionality may be embodied in whole or in part in firmware or hardware equivalents, such as integrated circuits, application-specific integrated circuits (ASICs), field programmable gate arrays (FPGA), and the like. Particular data structures may be used to more effectively implement one or more aspects of the disclosure, and such data structures are contemplated to be within the scope of computer executable instructions and computer-usable data described herein.

Various aspects described herein may be embodied as a method, an apparatus, or as one or more computer-readable media storing computer-executable instructions. Accordingly, those aspects may take the form of an entirely hardware embodiment, an entirely software embodiment, an entirely firmware embodiment, or an embodiment combining software, hardware, and firmware aspects in any combination. In addition, various signals representing data or events as described herein may be transferred between a source and a destination in the form of light or electromagnetic waves traveling through signal-conducting media such as metal wires, optical fibers, or wireless transmission media (e.g., air or space). In general, the one or more computer-readable media may be and/or include one or more non-transitory computer-readable media.

As described herein, the various methods and acts may be operative across one or more computing servers and one or more networks. The functionality may be distributed in any manner, or may be located in a single computing device (e.g., a server, a client computer, and the like). Additionally or alternatively, one or more of the computing platforms discussed above may be implemented in one or more virtual machines that are provided by one or more physical computing devices. In such arrangements, the various functions of each computing platform may be performed by the one or more virtual machines, and any and/or all of the above-discussed communications between computing platforms may correspond to data being accessed, moved, modified, updated, and/or otherwise used by the one or more virtual machines.

## Claims

1. A system comprising:
at least one processor; and
memory storing instructions that, when executed by the at least one processor, cause the system to:
receive, from a sequencing device, volumetric image data associated with a sample,
the volumetric image data comprising light intensity data;
identify, based on the volumetric image data, a set of temporally-ordered signals that represent a first sequence by clustering pixels of the volumetric image data;
identify, based on the set of temporally ordered signals, the first sequence located at first three-dimensional spatial coordinates; and
store, in a spatially searchable database, a first data element comprising the first three-dimensional spatial coordinates and a first identifier corresponding to the first sequence to spatially relate the first sequence to other sequences present in the sample.

2. The system of claim 1, wherein the memory stores additional instructions that, when executed by the at least one processor, cause the system to:
identify, based on the volumetric image data received from the sequencing device, a second sequence located at second three-dimensional spatial coordinates; and
store, in the spatially searchable database, a second data element comprising the second three-dimensional spatial coordinates and a second identifier corresponding to the second sequence to spatially relate the second sequence to the other sequences present in the sample.

3. The system of claim 1, wherein the memory stores additional instructions that, when executed by the at least one processor, cause the system to:
present, on a display device, information identifying a presence of the first sequence at the first three-dimensional spatial coordinates.

4. The system of claim 1, wherein the memory stores additional instructions that, when executed by the at least one processor, cause the system to:
determine one or more metrics associated with the first sequence at the first three-dimensional spatial coordinates; and
present, on a display device, information identifying the one or more metrics associated with the first sequence at the first three-dimensional spatial coordinates.

5. The system of claim 1, wherein the memory stores additional instructions that, when executed by the at least one processor, cause the system to:
perform a linked annotations query on the spatially searchable database to obtain annotation query results information; and
present, on a display device, at least a portion of the annotation query results information.

6. The system of claim 1, wherein the memory stores additional instructions that, when executed by the at least one processor, cause the system to:
perform a spatial query on the spatially searchable database to obtain spatial query results information; and
present, on a display device, at least a portion of the spatial query results information.

7. The system of claim 6, wherein the memory stores additional instructions that, when executed by the at least one processor, cause the system to:
receive user input requesting the spatial query, wherein the spatial query is performed in response to receiving the user input requesting the spatial query; or
(i) the spatial query on the spatially searchable database by jointly querying three-dimensional spatial coordinates data and linked annotations data maintained in the spatially searchable database,
(ii) the spatial query on the spatially searchable database by performing a three-dimensional distance query to identify a three-dimensional distance between the first sequence and another sequence present in the sample, or
(iii) the spatial query on the spatially searchable database by performing a containment query that determines whether the first sequence is contained within other features of the sample by identifying a position of the first sequence relative to the other features of the sample.

8. The system of claim 1, wherein the sequencing device is configured to sequence the sample using fluorescent in situ sequencing (FISSEQ), optionally wherein the volumetric image data received from the sequencing device comprises spatial information, temporal information, and color information associated with the sample.

9. A method implemented by a computer system comprising at least one processor and a memory, comprising:
using the at least one processor to receive, from a sequencing device, volumetric image data associated with a sample, said volumetric image data comprising light intensity data;
using the at least one processor to identify, based on the volumetric image data, a set of temporally-ordered signals that represent a first sequence by clustering pixels of the volumetric image data;
using the at least one processor to identify, based on the set of temporally-ordered signals, the first sequence located at first three-dimensional spatial coordinates; and
using the at least one processor to store, in a spatially searchable database, a first data element comprising the first three-dimensional spatial coordinates and a first identifier corresponding to the first sequence to spatially relate the first sequence to other sequences present in the sample.

10. The method of claim 9, further comprising:
using the at least one processor to identify, based on the volumetric image data received from the sequencing device, a second sequence located at second three-dimensional spatial coordinates; and
using the at least one processor to store, in the spatially searchable database, a second data element comprising the second three-dimensional spatial coordinates and a second identifier corresponding to the second sequence to spatially relate the second sequence to the other sequences present in the sample.

11. The method of claim 9, further comprising:
using the at least one processor to present, on a display device, information identifying a presence of the first sequence at the first three-dimensional spatial coordinates; or
using the at least one processor to perform a linked annotations query on the spatially searchable database to obtain annotation query results information; and using the at least one processor to present, on a display device, at least a portion of the annotation query results information; or
using the at least one processor to perform a spatial query on the spatially searchable database to obtain spatial query results information; and using the at least one processor to present, on a display device, at least a portion of the spatial query results information.

12. The method of claim 9, wherein the sequencing device sequences the sample using fluorescent in situ sequencing (FISSEQ), optionally wherein the volumetric image data received from the sequencing device comprises spatial information, temporal information, and color information associated with the sample.

13. The system of claim 1, wherein the light intensity data is obtained from optically encoded nucleic acid sequencing methods.

14. The system of claim 1, wherein the first data element further comprises a feature set including biological annotations, cellular annotations or morphological annotations.

15. The method of claim 9, wherein the light intensity data is obtained from optically encoded nucleic acid sequencing methods.

16. The method of claim 9, wherein the first data element further comprises a feature set including biological annotations, cellular annotations or morphological annotations.

## Patentansprüche

1. System, Folgendes aufweisend:
zumindest einen Prozessor und
einen Speicher, der Anweisungen speichert, die, wenn sie vom zumindest einem Prozessor ausgeführt werden, das System veranlassen:
von einer Sequenzierungsvorrichtung volumetrische Bilddaten zu erhalten, die einer Probe zugeordnet sind, wobei die volumetrischen Bilddaten Daten zur Lichtintensität umfassen;
basierend auf den volumetrischen Bilddaten einen Satz zeitlich geordneter Signale zu identifizieren, die mittels Clustering von Pixeln der volumetrischen Bilddaten eine erste Sequenz darstellen;
basierend auf dem Satz zeitlich geordneter Signale die erste Sequenz zu identifizieren, die an ersten dreidimensionalen Raumkoordinaten angeordnet ist; und
in einer räumlich durchsuchbaren Datenbank ein erstes Datenelement zu speichern, das die ersten dreidimensionalen Raumkoordinaten und eine erste Kennung umfasst, die der ersten Sequenz entspricht, um die erste Sequenz mit anderen Sequenzen, die in der Probe vorliegen, räumlich miteinander in Beziehung zu setzen.

2. System nach Anspruch 1, wobei der Speicher zusätzliche Anweisungen speichert, die, wenn sie durch den zumindest einen Prozessor ausgeführt werden, das System dazu veranlassen:
basierend auf den von der Sequenzierungsvorrichtung erhaltenen volumetrischen Bilddaten eine zweite Sequenz zu identifizieren, die an zweiten dreidimensionalen Raumkoordinaten angeordnet ist; und
in der räumlich durchsuchbaren Datenbank ein zweites Datenelement zu speichern, das die zweiten dreidimensionalen Raumkoordinaten und eine zweite Kennung umfasst, die der zweiten Sequenz entspricht, um die zweite Sequenz mit den anderen Sequenzen, die in der Probe vorliegen sind, räumlich in Beziehung zu setzen.

3. System nach Anspruch 1, wobei der Speicher zusätzliche Anweisungen speichert, die, wenn sie durch den zumindest einen Prozessor ausgeführt werden, das System dazu veranlassen:
auf einer Anzeigevorrichtung Informationen darzustellen, die ein Vorliegen der ersten Sequenz an den ersten dreidimensionalen Raumkoordinaten erkennen.

4. System nach Anspruch 1, wobei der Speicher zusätzliche Anweisungen speichert, die, wenn sie durch den zumindest einen Prozessor ausgeführt werden, das System dazu veranlassen:
eine oder mehrere Metriken, die der ersten Sequenz an den ersten dreidimensionalen Raumkoordinaten zugeordnet sind, zu ermitteln; und
auf einer Anzeigevorrichtung Informationen darzustellen, die die eine oder mehreren Metriken, die der ersten Sequenz zugeordnet ist bzw. sind, an den ersten dreidimensionalen Raumkoordinaten identifizieren.

5. System nach Anspruch 1, wobei der Speicher zusätzliche Anweisungen speichert, die, wenn sie durch den zumindest einen Prozessor ausgeführt werden, das System dazu veranlassen:
eine Abfrage mit verknüpften Annotationen in der räumlich durchsuchbaren Datenbank auszuführen, um Ergebnisinformationen der Abfrage mit verknüpften Annotationen zu erhalten; und
auf einer Anzeigevorrichtung zumindest ein Teil der Ergebnisinformationen der Annotations-Abfrage darzustellen.

6. System nach Anspruch 1, wobei der Speicher zusätzliche Anweisungen speichert, die, wenn sie durch den zumindest einen Prozessor ausgeführt werden, das System dazu veranlassen:
eine räumliche Abfrage in der räumlich durchsuchbaren Datenbank auszuführen, um Ergebnisinformationen der räumlichen Abfrage zu erhalten; und
auf einer Anzeigevorrichtung zumindest einen Teil der Ergebnisinformationen der räumlichen Abfrage darzustellen.

7. System nach Anspruch 6, wobei der Speicher zusätzliche Anweisungen speichert, die, wenn sie durch den zumindest einen Prozessor ausgeführt werden, das System dazu veranlassen:
eine Benutzereingabe zu erhalten, die die räumliche Abfrage anfordert, wobei die räumliche Abfrage als Reaktion auf den Erhalt der Benutzereingabe, die die räumliche Abfrage anfordert, ausgeführt wird; oder
(i) die räumliche Abfrage in der räumlich durchsuchbaren Datenbank durch gemeinsames Abfragen von dreidimensionalen Raumkoordinatendaten und Daten mit verknüpften Annotationen, die in der räumlich durchsuchbaren Datenbank geführt werden,
(ii) die räumliche Abfrage in der räumlich durchsuchbaren Datenbank durch Ausführen einer Abfrage bezüglich des dreidimensionalen Abstands, um einen dreidimensionalen Abstand zwischen der ersten Sequenz und einer anderen in der Probe vorliegenden Sequenz zu erkennen, oder
(iii) die räumliche Abfrage in der räumlich durchsuchbaren Datenbank durch Ausführen einer Containment-Abfrage, die ermittelt, ob die erste Sequenz in anderen Merkmalen der Probe enthalten ist, indem sie eine Position der ersten Sequenz in Bezug auf die anderen Merkmale der Probe identifiziert.

8. System nach Anspruch 1, wobei die Sequenzierungsvorrichtung so ausgebildet ist, dass sie die Probe mittels Fluoreszenz-insitu-Sequenzierung (FISSEQ) sequenziert, wobei die von der Sequenzierungsvorrichtung erhaltenen volumetrischen Bilddaten optional räumliche Informationen, zeitliche Informationen und Farbinformationen, die der Probe zugeordnet sind, umfassen.

9. Verfahren, das durch ein Computersystem implementiert wird, das zumindest einen Prozessor und einen Speicher umfasst, Folgendes umfassend:
Verwenden des zumindest einen Prozessors, um von einer Sequenzierungsvorrichtung volumetrische Bilddaten zu erhalten, die einer Probe zugeordnet sind, wobei die volumetrischen Bilddaten Daten zur Lichtintensität umfassen;
Verwenden des zumindest einen Prozessors, um basierend auf den volumetrischen Bilddaten einen Satz zeitlich geordneter Signale zu identifizieren, die mittels Clustering von Pixeln der volumetrischen Bilddaten eine erste Sequenz darstellen;
Verwenden des zumindest einen Prozessors, um basierend auf dem Satz zeitlich geordneter Signale die erste Sequenz zu identifizieren, die an ersten dreidimensionalen Raumkoordinaten angeordnet ist; und
Verwenden des zumindest einen Prozessors, um in einer räumlich durchsuchbaren Datenbank ein erstes Datenelement zu speichern, das die ersten dreidimensionalen Raumkoordinaten und eine erste Kennung umfasst, die der ersten Sequenz entspricht, um die erste Sequenz mit anderen Sequenzen, die in der Probe vorliegen, räumlich in Beziehung zu setzen.

10. Verfahren nach Anspruch 9, ferner Folgendes umfassend:
Verwenden des zumindest einen Prozessors, um basierend auf den von der Sequenzierungsvorrichtung erhaltenen volumetrischen Bilddaten eine zweite Sequenz zu identifizieren, die an zweiten dreidimensionalen Raumkoordinaten angeordnet ist; und
Verwenden des zumindest einen Prozessors, um in der räumlich durchsuchbaren Datenbank ein zweites Datenelement zu speichern, das die zweiten dreidimensionalen Raumkoordinaten und eine zweite Kennung umfasst, die der zweiten Sequenz entspricht, um die zweite Sequenz mit den anderen Sequenzen, die in der Probe vorliegen, räumlich in Beziehung zu setzen.

11. Verfahren nach Anspruch 9, ferner Folgendes umfassend:
Verwenden des zumindest einen Prozessors, um auf einer Anzeigevorrichtung Informationen darzustellen, die ein Vorliegen der ersten Sequenz an den ersten dreidimensionalen Raumkoordinaten erkennen; oder
Verwenden des zumindest einen Prozessors, um eine Abfrage mit verknüpfte Annotationen in der räumlich durchsuchbaren Datenbank auszuführen, um Ergebnisinformationen der Abfrage mit verknüpften Annotationen zu erhalten; und Verwenden des zumindest einen Prozessors, um auf einer Anzeigevorrichtung zumindest einen Teil der Ergebnisinformationen der Abfrage mit verknüpften Annotationen darzustellen; oder
Verwenden des zumindest einen Prozessors, um eine räumliche Abfrage in der räumlich durchsuchbaren Datenbank auszuführen, um Ergebnisinformationen der räumlichen Abfrage zu erhalten; Verwenden des zumindest einen Prozessors, um auf einer Anzeigevorrichtung zumindest einen Teil der Ergebnisinformationen der räumlichen Abfrage darzustellen.

12. Verfahren nach Anspruch 9, wobei die Sequenzierungsvorrichtung die Probe mittels Fluoreszenz-insitu-Sequenzierung (FISSEQ) sequenziert, wobei die von der Sequenzierungsvorrichtung erhaltenen volumetrischen Bilddaten optional räumliche Informationen, zeitliche Informationen und Farbinformationen umfassen, die der Probe zugeordnet sind.

13. System nach Anspruch 1, wobei die Daten zur Lichtintensität aus Verfahren optisch codierter Nukleinsäuresequenzierung erhalten werden.

14. System nach Anspruch 1, wobei das erste Datenelement ferner einen Merkmalssatz umfasst, der biologische Annotationen, zelluläre Annotationen oder morphologische Annotationen umfasst.

15. Verfahren nach Anspruch 9, wobei die Daten zur Lichtintensität aus Verfahren optisch codierter Nukleinsäuresequenzierung erhalten werden.

16. Verfahren nach Anspruch 9, wobei das erste Datenelement ferner einen Merkmalssatz umfasst, der biologische Annotationen, zelluläre Annotationen oder morphologische Annotationen umfasst.

## Revendications

1. Système comprenant :
au moins un processeur ; et
la mémoire stockant des instructions qui, lorsqu'elles sont exécutées par au moins un processeur, amènent le système à :
recevoir, à partir d'un dispositif de séquençage, des données d'image volumétriques associées à un échantillon, les données d'image volumétriques comprenant des données d'intensité lumineuse ;
identifier, sur la base des données d'image volumétriques, un ensemble de signaux ordonnés temporellement qui représentent une première séquence en regroupant des pixels des données d'image volumétriques ;
identifier, sur la base de l'ensemble de signaux ordonnés temporellement, la première séquence située au niveau de premières coordonnées spatiales tridimensionnelles ; et
stocker, dans une base de données spatialement consultable, un premier élément de données comprenant les premières coordonnées spatiales tridimensionnelles et un premier identifiant correspondant à la première séquence pour lier spatialement la première séquence à d'autres séquences présentes dans l'échantillon.

2. Système selon la revendication 1, dans lequel la mémoire stocke des instructions supplémentaires qui, lorsqu'elles sont exécutées par l'au moins un processeur, amènent le système à :
identifier, sur la base des données d'image volumétriques reçues du dispositif de séquençage, une deuxième séquence située au niveau de deuxièmes coordonnées spatiales tridimensionnelles ; et
stocker, dans la base de données spatialement consultable, un deuxième élément de données comprenant les deuxièmes coordonnées spatiales tridimensionnelles et un deuxième identifiant correspondant à la deuxième séquence pour lier spatialement la deuxième séquence aux autres séquences présentes dans l'échantillon.

3. Système selon la revendication 1, dans lequel la mémoire stocke des instructions supplémentaires qui, lorsqu'elles sont exécutées par l'au moins un processeur, amènent le système à :
présenter, sur un dispositif d'affichage, des informations identifiant une présence de la première séquence aux premières coordonnées spatiales tridimensionnelles.

4. Système selon la revendication 1, dans lequel la mémoire stocke des instructions supplémentaires qui, lorsqu'elles sont exécutées par l'au moins un processeur, amènent le système à :
déterminer une ou plusieurs métriques associées à la première séquence aux premières coordonnées spatiales tridimensionnelles ; et
présenter, sur un dispositif d'affichage, des informations identifiant les une ou plusieurs métriques associées à la première séquence aux premières coordonnées spatiales tridimensionnelles.

5. Système selon la revendication 1, dans lequel la mémoire stocke des instructions supplémentaires qui, lorsqu'elles sont exécutées par l'au moins un processeur, amènent le système à :
réaliser une interrogation d'annotation liée sur la base de données spatialement consultable pour obtenir des informations de résultats d'interrogation d'annotation ; et
présenter, sur un dispositif d'affichage, au moins une portion des informations de résultats d'interrogation d'annotation.

6. Système selon la revendication 1, dans lequel la mémoire stocke des instructions supplémentaires qui, lorsqu'elles sont exécutées par l'au moins un processeur, amènent le système à :
réaliser une interrogation spatiale sur la base de données spatialement consultable pour obtenir des informations de résultats d'interrogation spatiale ; et
présenter, sur un dispositif d'affichage, au moins une portion des informations de résultats d'interrogation spatiale.

7. Système selon la revendication 6, dans lequel la mémoire stocke des instructions supplémentaires qui, lorsqu'elles sont exécutées par l'au moins un processeur, amènent le système à :
recevoir une entrée utilisateur demandant l'interrogation spatiale, dans lequel l'interrogation spatiale est réalisée en réponse à la réception de l'entrée utilisateur demandant l'interrogation spatiale ; ou
(i) l'interrogation spatiale sur la base de données spatialement consultable en interrogeant conjointement des données de coordonnées spatiales tridimensionnelles et des données d'annotations liées conservées dans la base de données spatialement consultable,
(ii) l'interrogation spatiale sur la base de données spatialement consultable en réalisant une interrogation de distance tridimensionnelle pour identifier une distance tridimensionnelle entre la première séquence et une autre séquence présente dans l'échantillon, ou
(iii) l'interrogation spatiale sur la base de données spatialement consultable en réalisant une interrogation de confinement qui détermine si la première séquence est contenue au sein d'autres caractéristiques de l'échantillon en identifiant une position de la première séquence par rapport aux autres caractéristiques de l'échantillon.

8. Système selon la revendication 1, dans lequel le dispositif de séquençage est configuré pour séquencer l'échantillon en utilisant un séquençage in situ fluorescent (FISSEQ), facultativement dans lequel les données d'image volumétriques reçues depuis le dispositif de séquençage comprennent des informations spatiales, des informations temporelles et des informations de couleur associées à l'échantillon.

9. Procédé mis en œuvre par un système informatique comprenant au moins un processeur et une mémoire, comprenant :
l'utilisation d'au moins un processeur pour recevoir, à partir d'un dispositif de séquençage, des données d'image volumétriques associées à un échantillon, lesdites données d'image volumétriques comprenant des données d'intensité lumineuse ;
l'utilisation d'au moins un processeur pour identifier, sur la base des données d'image volumétriques, un ensemble de signaux ordonnés temporellement qui représentent une première séquence en regroupant des pixels des données d'image volumétriques ;
l'utilisation d'au moins un processeur pour identifier, sur la base de l'ensemble de signaux ordonnés temporellement, la première séquence située au niveau de premières coordonnées spatiales tridimensionnelles ; et
l'utilisation d'au moins un processeur pour stocker, dans une base de données spatialement consultable, un premier élément de données comprenant les premières coordonnées spatiales tridimensionnelles et un premier identifiant correspondant à la première séquence pour lier spatialement la première séquence à d'autres séquences présentes dans l'échantillon.

10. Procédé selon la revendication 9, comprenant en outre :
l'utilisation d'au moins un processeur pour identifier, sur la base des données d'image volumétriques reçues du dispositif de séquençage, une deuxième séquence située au niveau de deuxièmes coordonnées spatiales tridimensionnelles ; et
l'utilisation d'au moins un processeur pour stocker, dans la base de données spatialement consultable, un deuxième élément de données comprenant les deuxièmes coordonnées spatiales tridimensionnelles et un deuxième identifiant correspondant à la deuxième séquence pour lier spatialement la deuxième séquence aux autres séquences présentes dans l'échantillon.

11. Procédé selon la revendication 9, comprenant en outre :
l'utilisation d'au moins un processeur pour présenter, sur un dispositif d'affichage, des informations identifiant une présence de la première séquence aux premières coordonnées spatiales tridimensionnelles ; ou
l'utilisation d'au moins un processeur pour réaliser une interrogation d'annotation liée sur la base de données spatialement consultable pour obtenir des informations de résultats d'interrogation d'annotation ; l'utilisation d'au moins un processeur pour présenter, sur un dispositif d'affichage, au moins une portion des informations de résultats d'interrogation d'annotation ; ou
l'utilisation d'au moins un processeur pour réaliser une interrogation spatiale sur la base de données spatialement consultable pour obtenir des informations de résultats d'interrogation spatiale ; l'utilisation d'au moins un processeur pour présenter, sur un dispositif d'affichage, au moins une portion des informations de résultats d'interrogation spatiale.

12. Procédé selon la revendication 9, dans lequel le dispositif de séquençage séquence l'échantillon en utilisant un séquençage in situ fluorescent (FISSEQ), facultativement dans lequel les données d'image volumétriques reçues depuis le dispositif de séquençage comprennent des informations spatiales, des informations temporelles et des informations de couleur associées à l'échantillon.

13. Système selon la revendication 1, dans lequel les données d'intensité lumineuse sont obtenues à partir de procédés de séquençage d'acides nucléiques codés optiquement.

14. Système selon la revendication 1, dans lequel le premier élément de données comprend en outre un ensemble de caractéristiques comportant des annotations biologiques, des annotations cellulaires ou des annotations morphologiques.

15. Procédé selon la revendication 9, dans lequel les données d'intensité lumineuse sont obtenues à partir de procédés de séquençage d'acides nucléiques codés optiquement.

16. Procédé selon la revendication 9, dans lequel le premier élément de données comprend en outre un ensemble de caractéristiques comportant des annotations biologiques, des annotations cellulaires ou des annotations morphologiques.
